# EUROPEAN PATENT APPLICATION

(11) **EP 4 481 057 A1**
(43) Date of publication of application: **25.12.2024**
(21) Application number: 23305977.3
(22) Date of filing: 20.06.2023
(51) Int. Cl.: C12Q 1/6813

(54) **SAMPLE PREPARATION METHODS USING MODIFIED BASES FOR RAPID IDENTIFICATION AND SEQUENCING**

(71) Applicant: Depixus, 75014 Paris (FR)
(72) Inventor: OUELLET, Jimmy, 91510 LARDY (FR); MALUENDA, Jérôme, 91220 BRETIGNY (FR); D'AVEZAC, Pol, 92160 ANTONY (FR); ANDRE, Charles, SAN FRANCISCO, 94114 (US)
(74) Representative: Regimbeau

(57) **Abstract**

The present invention provides a method for preparing a nucleic acid sample for producing hairpin molecules, preferably from small dsDNA molecules, notably from cfDNA, or from small RNA molecules such as fragmented RNA molecules, in order to identify them rapidly on nucleic acid analysis instruments (e.g., the MAGNA platform) with a minimal set of reagents.

## Description

### INTRODUCTION

The present invention provides a method for preparing a nucleic acid sample for producing hairpin molecules, preferably from small dsDNA molecules, notably from cfDNA, or from small RNA molecules such as fragmented RNA molecules, in order to identify them rapidly on nucleic acid analysis instruments (e.g., the MAGNA platform) with a minimal set of reagents.

There are many situations when the quantity of nucleic acid (typically DNA or RNA) available to perform a genetic or epigenetic analysis is limited. Sample types include tumour biopsies, anthropology specimens, and forensic specimens. Amplification is usually performed to increase the amount of starting material. Some major disadvantages, however, are associated with amplification. In particular, the epigenetic modifications are not conserved throughout the amplification process. In addition, amplification shows variable efficiency depending on the number and sequence of the different targets being simultaneously analysed.

The present inventors previously designed a single-molecule analysis method (Ding et al. Nat. Methods 9: 367-372, 2012) which can decode many new layers of dynamic genetic information held within nucleic acids with very high accuracy and without need for sample amplification (Wang et al. Commun Biol 4: 128, 2021). Determination of a nucleic acid sequence, as well as a number of related applications, including DNA detection and quantification, detection of protein binding to nucleic acids, and identification of epigenetic base modifications have notably been achieved (WO 2011/147931; WO 2011/147929; WO 2013/093005; WO 2014/114687).

According to this method, a single nucleic acid hairpin is denatured by pulling on its extremities e.g., with magnetic tweezers. Reducing the tension below a threshold allows for the renaturation of the hairpin. However, if the denatured hairpin has been hybridised to a single-stranded oligonucleotide, then renaturation will be blocked, at least transiently. The double-stranded molecule can then be identified by determining the duration and/or location of the blockage.

For the identification of random DNA or RNA fragments, this technology relies on the binding pattern of small oligonucleotides (of 3 or 4 bases) that create a specific pattern or barcode. This experimental barcode can then be compared to the sequenced reference genome from which this fragment was generated and matched the expected pattern. This strategy works well if the fragments to be identified are long enough such that the density of binding of small oligonucleotides is dense enough to create a unique barcode.

However, using this approach for analysing short nucleic acid fragments (< 200 bp), is challenging. For these short fragments, the density is not sufficient to create unique signature (unless a large portion of trimers is used) and therefore lead to ambiguous solutions (the fragments are mapped to multiple regions of the genome, especially for large genomes like it is the case for human). Moreover, epigenetic modifications must be preserved, since it is important to be able to detect a variety of genetic, epigenetic, and base modification changes concomitantly within the same single molecules. Finally, it is important to use starting material in amounts as little as possible.

There is thus still a need for a simple and reliable method for identifying short nucleic acid fragments.

### SUMMARY OF THE DISCLOSURE

The present invention relates to the identification of small nucleic acid molecules. It is based on the use of modified nucleotides to specifically distinguish two strands of nucleic acid. The detection of the nucleotide analogue in the nucleic acid generates a unique signature which can be used to identify univocally the small nucleic acid molecule of interest. Moreover, as the modified bases used are not present in the native nucleic acid, epigenetic modifications can be detected, thus adding new layers of information obtained through the same method.

In a first aspect, the present disclosure provides a method of identifying a nucleic acid molecule in a population of nucleic acid molecules, the method comprising replicating the nucleic acid molecule with a polymerase in the presence of a modified nucleotide triphosphate, and detecting the position of the modified nucleotide incorporated in the newly synthesised nucleic acid molecule.

More specifically, the method disclosed herein comprises the steps of:
a) contacting a nucleic acid molecule with a polymerase and a mix of nucleotides triphosphates, wherein at least one of the nucleotides triphosphates is a modified nucleotide triphosphate,
b) performing a replication reaction with the polymerase and the nucleotides triphosphates mix of step a), and with the nucleic acid molecule as template,
c) detecting the position of the modified nucleotide in the nucleic acid molecules obtained by the reaction of step b), thereby generating a unique signature, and
d) identifying the nucleic acid molecule based on the unique signature of step c).

Preferably, the nucleic acid molecule is less than 500 nucleotides long, preferably less than 400 nucleotides, preferably less than 300 nucleotides, more preferably less than 200 nucleotides.

Preferably, the population of nucleic acid molecules is a population of circulating nucleic acid molecules.

Preferably, the modified nucleotide is selected in the group consisting of nucleotides incorporating bases such as 3-nitropyrrole 2'-deoxynucloside and 5-nitroindole 2'-deoxynucleoside, alpha phosphorothiolate, phosphorothioate nucleotide triphosphates, pyrazolo[3,4-d]pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyl uracil and cytosine, 6-azo uracil, cytosine and thymine, 5-uracil (pseudouracil), 4-thiouracil, 8-halo (e.g., 8-bromo), 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, deazaguanine, 7-deazaguanine, 3-deazaguanine, deazaadenine, 7-deazaadenine, 3-deazaadenine, pyrazolo[3,4-d]pyrimidine, imidazo[1,5-a]1,3,5 triazinones, 9-deazapurines, imidazo[4,5-d]pyrazines, thiazolo[4,5-d]pyrimidines, pyrazin-2-ones, 1,2,4-triazine, pyridazine; and 1,3,5 triazine; the modified nucleotide being preferably selected in the group consisting of 5-bromo-2'-deoxyuridine-5'-triphosphate, 5-iodo-2'-deoxyuridine-5'-triphosphate, 5-fluoro-2'-deoxyuridine-5'-triphosphate, 5-ethynyl-2'-deoxyuridine-5'-triphosphate, 5-chloro-2'-deoxyuridine-5'-triphosphate, 5-methyluridine triphosphate, 5-hydroxymethyluridine-5'-triphosphate, 1-methyl-pseudouridine-5-triphosphate, 1-methyl-pseudouridine-phosphoramidite, pseudouridine-5-triphosphate, 5-formyl-2'-deoxyuridine-5'-triphosphate, 5-carboxy-2'-deoxyuridine-5'-triphosphate, 5-fluorouridine-5'-triphosphate; 5-methyluridine (m⁵U), 5-taurinomethyluridine (τm⁵U), 5-carboxymethylaminomethyluridine (cmnm⁵U), 5-hydroxyuridine (ho⁵U), 5-methoxyuridine (mo⁵U), 5-carboxymethyluridine (cmo⁵U), 5-carboxymethoxyuridine (mcmo⁵U), 5-bromo-2'-deoxycytidine-5' triphosphate, 5 iodo 2' deoxycytidine 5' triphosphate, 5 fluoro 2' deoxycytidine 5' triphosphate; the modified nucleotide being more preferably in the group consisting of 5-bromo-2'-deoxyuridine-5'-triphosphate, 5-iodo-2'-deoxyuridine-5'-triphosphate, 5-fluoro-2'-deoxyuridine-5'-triphosphate, pseudouridine-5'-triphosphate, 5-formyl-2'-deoxyuridine-5'-triphosphate, 5-carboxy-2'-deoxyuridine-5'-triphosphate, 5-hydroxymethyl-2'-deoxyuridine-5'-triphosphate, and 5-chloro-2'-deoxyuridine-5'-triphosphate.

In another preferred instance, the polymerase of step a) is selected in the group consisting of: Φ29 DNA polymerase, Bst DNA Polymerase, DeepVent, Manta, OmniAmp polymerase, SD polymerase, Bst DNA Polymerase Large Fragment, Bst 2.0 DNA Polymerase, Bst 3.0 DNA Polymerase, and Bsu DNA Polymerase Large Fragment.

Preferably, the method comprises a further step of detecting epigenetic modifications, more preferably 5-methylcytosine (5mC), 5-hydroxymethylcytosine (5hmC), 5-formylcytosine (5fC) 5-carboxylcytosine (5caC), 5-hydroxymethyluracil (5hmU), and N6-methyladenosine (m6A), even more preferably 5mC and 5hmC.

In a preferred instance, a hairpin is ligated prior to step a) to at least one end of the nucleic acid molecule of interest. More preferably, the hairpin is nicked. Preferably, the hairpin comprises at most 10, 9, 8, 7, 6, or 5 nucleotides. More preferably, the hairpin loop consists of 3, 4, or 5 nucleotides. Even more preferably, the loop consists of 4 bases. Furthermore, the hairpin comprises a double-stranded stem comprising at most 100 bp, 90 bp, 80 bp, 70 bp, 60 bp, 50 bp, 40 bp, 30 bp, 20 bp, 15 bp, or 10 bp. More preferably, the double-stranded stem consists of 8, 15, 20, 40, or 60 bp.

Another preferred instance comprises ligating a second hairpin, identical to or different from, the first hairpin, to the second end of the nucleic acid molecule, prior to step a). When the second hairpin is different, the hairpin preferably comprises at most 50, 45, 40, 35, 30, or 25 nucleotides. More preferably, the hairpin loop consists of 10, 15, or 20 nucleotides. Even more preferably, the loop consists of 15 bases. Furthermore, the hairpin comprises a double-stranded stem comprising at most 100 bp, 90 bp, 80 bp, 70 bp, 60 bp, 50 bp, 40 bp, 30 bp, 20 bp, 15 bp, or 10 bp. More preferably, the double-stranded stem consists of 8, 15, 20, 40, or 60 bp.

In another preferred instance, the loop of the first hairpin and/or the loop of the second hairpin does not comprise a nucleotide complementary to the modified nucleotide triphosphate.

In another instance, step c) of the method disclosed herein comprises contacting the nucleic acid with a protein binding specifically the modified nucleotide triphosphate. Preferably, the protein is an antibody specific for the modified nucleotide triphosphate.

In another instance, detection of an epigenetic modification, e.g., a modified base, comprises contacting the nucleic acid with a protein binding specifically the epigenetic modification. Preferably, the protein is an antibody specific for the epigenetic modification.

In another aspect, detection of the position of the modified nucleotide comprises detecting the binding to the modified nucleotide of the protein binding specifically to this nucleotide analogue. Preferably, the nucleotide analogues are detected in the replicated strand using the technology developed by the present inventors, i.e., the so-called MAGNA^{™} technology (Ding et al. Nat. Methods 9: 367-372, 2012; Wang et al. Commun Biol 4: 128, 2021; WO 2011/147931; WO 2011/147929; WO 2013/093005; WO 2014/114687).

In a preferred instance, the detection of the binding of the protein to the modified nucleotide comprises the steps of:
(i) denaturing the replicated double-stranded nucleic acid molecule by applying a physical force to the said molecule;
(ii) providing the protein capable of binding specifically to the modified nucleotide;
(iii) renaturing the double stranded nucleic acid molecule in the presence of the protein;
(iv) detecting a blockage of the renaturation of the double-stranded nucleic acid; and
(v) determining the position of the blockage on said double-stranded nucleic acid molecule.

Preferably, at least one of the bases of one of the strands of the double-stranded nucleic acid is attached directly or indirectly to a support, and wherein at least one of the bases of the other strand of the double-stranded nucleic acid is attached to a movable support.

Preferably, the double-stranded nucleic acid is denatured in step (i) by moving away the supports. More preferably, a physical force above or equal to 15 pN, preferably above or equal to 17 pN, more preferably above or equal to 18 pN, is applied to the double-stranded nucleic acid molecule by moving away the supports.

Preferably, the denatured double-stranded nucleic acid is renatured in step (iii) by bringing the supports together. More preferably, the force applied to the double-stranded molecule is reduced to less than or equal to 12 pN, preferably less than or equal to 11 pN, more preferably less than or equal to 10 pN, by bringing the supports together.

In another preferred instance, steps (i) to (iv) are repeated several times (so as to accumulate measurements and increase the signal/noise ratio).

Preferably, the detection of step (iv) comprises measuring the distance (z) between the two ends of the double-stranded nucleic acid molecule which are attached to the support. More preferably, the detection of step (iv) comprises a further step of measuring the distance (z_{high}) between the two ends of the double-stranded nucleic acid molecules which are attached to the support, when the double-stranded nucleic acid molecule is denatured. Even more preferably, step (v) further comprises the prior step of comparing z and z_{high}.

In another preferred instance, detection of the binding of the protein to the modified nucleotide comprises a further step of measuring the duration of the blockage. More preferably, it comprises a further step of comparing the duration of the blockage with a reference value.

Another aspect of the present disclosure relates to a method of quantifying a nucleic acid molecule in a population of nucleic acids comprising the steps of:
α) identifying the nucleic acid molecule using the method described above; and
β) numerating the nucleic acid molecule identified in step α).

Another aspect of the present disclosure relates to a method of detecting a nucleic acid molecule of foetal origin in a biological sample from a pregnant female, wherein the method comprises:
A. detecting the presence of the nucleic acid molecule in the sample using the method described above,
wherein the nucleic acid molecule is a paternally inherited sequence which is not possessed by the pregnant female.

Another aspect of the present disclosure relates to a method of detecting foetal aneuploidy in a population of nucleic acid molecules, wherein the population comprises maternal and foetal genetic material, in a biological sample from a pregnant female, comprising the steps of:
A. quantifying a nucleic acid molecule comprising a signature associated with foetal aneuploidy using the method described above;
B. quantifying a reference nucleic acid molecule, the reference nucleic acid molecule being preferably a nucleic acid molecule comprising a signature not associated with foetal aneuploidy, using the method described above; and
C. comparing the quantifications of steps A and B;
wherein foetal aneuploidy is indicated by a number of molecules in A higher than the number of molecules in B. Preferably, the number of molecules in A and the number of molecules in B are normalised to the total number of molecules.

Another aspect of the present disclosure relates to a method of diagnosing a tumour in a patient, wherein the method comprises:
A. detecting the presence of a nucleic acid molecule in a sample from the patient using the method described above,
wherein the nucleic acid molecule comprises a tumour-specific signature.

Preferably, the method further comprises the step of:
B. quantifying a nucleic acid molecule comprising a tumour-specific sequence using the method described above;
wherein the presence of a tumour is indicated by a number of molecules in A.

Preferably, the method further comprises the steps of:
C. quantifying a reference nucleic acid molecule using the method disclosed above; and
D. comparing the quantifications of steps A and B;
wherein tumour aneuploidy is indicated by a number of molecules in A higher than the number of molecules in B. More preferably, the number of molecules in A and the number of molecules in B are normalised to the total number of molecules.

Preferably, the biological sample of the methods above is a sample selected in the group consisting of body fluids, including but not limited to blood, plasma, serum, mucus, lymphatic fluid, synovial fluid, cerebrospinal fluid, saliva, amniotic fluid, amniotic cord blood, urine, vaginal fluid and semen; preferably blood, serum and plasma; more preferably, blood.

In another aspect, the disclosure provides a kit for protecting the methods described herein. Preferably, the kit according to the disclosure comprises:
- a mix of nucleotides triphosphates, wherein at least one of the nucleotides triphosphates is a modified nucleotide triphosphate as described herein;
- at least one polymerase as described herein;
- optionally, at least one hairpin as described herein; and
- optionally, one protein binding to the modified nucleotide.

Preferably, the kit disclosed herein further comprises a double-stranded spacer molecule comprising two complementary, self-annealing sequences bordered by two single-stranded sequences.

### FIGURE LEGENDS

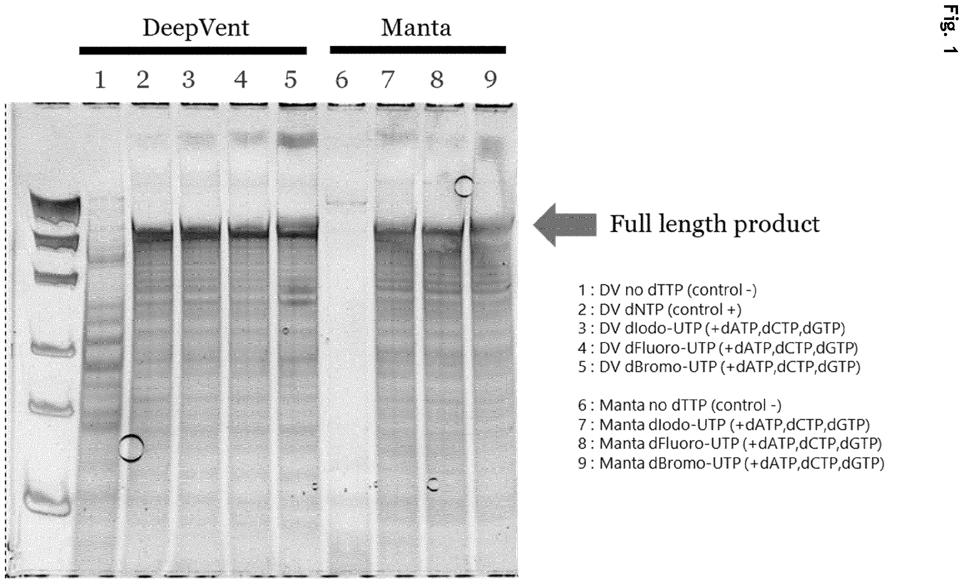

**Figure 1****: Ability of DNA polymerase to integrate modified uridine triphosphate.**
   A synthetic template of 200 bases was used to determine the capacity of Deep Vent and Manta DNA polymerase to integrate modified uridine triphosphate. The synthesis was initiated from a 20 bases complementary oligonucleotide and various conditions were tested. In lanes 1 and 6, all triphosphate nucleotide except dTTP were added in the reaction mix according to the manufacturer recommendation (negative control) to determine if the polymerase is stopped at positions where it needs to integrate a thymidine. In lane 2, dTTP was added to the reaction mix as the positive control where full length construct should be obtained. In lanes 3 and 7, 4 and 8 and 5 and 9, IdU, FdU and CldU were added to the reaction mix respectively to replace at 100% the dTTP. For both DNA polymerases, full length products were obtained with all three modified nucleotides. However, we noticed some intermediate products in the condition where dTTP was omitted for the DeepVent DNA polymerase, which suggests that the polymerase may integrate the wrong nucleotide at these positions. This is not the case for the Manta DNA polymerase as the only product observed is where the polymerase should have integrated a thymidine.

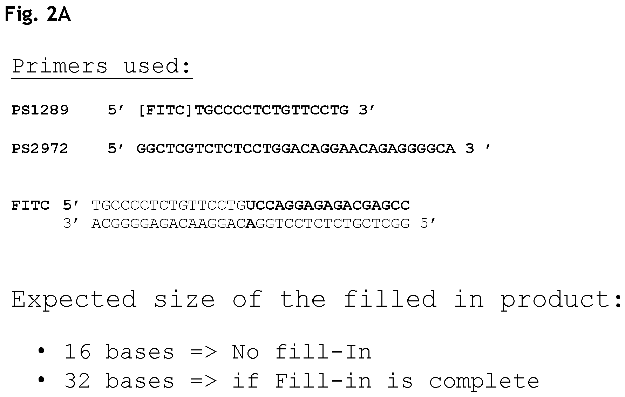

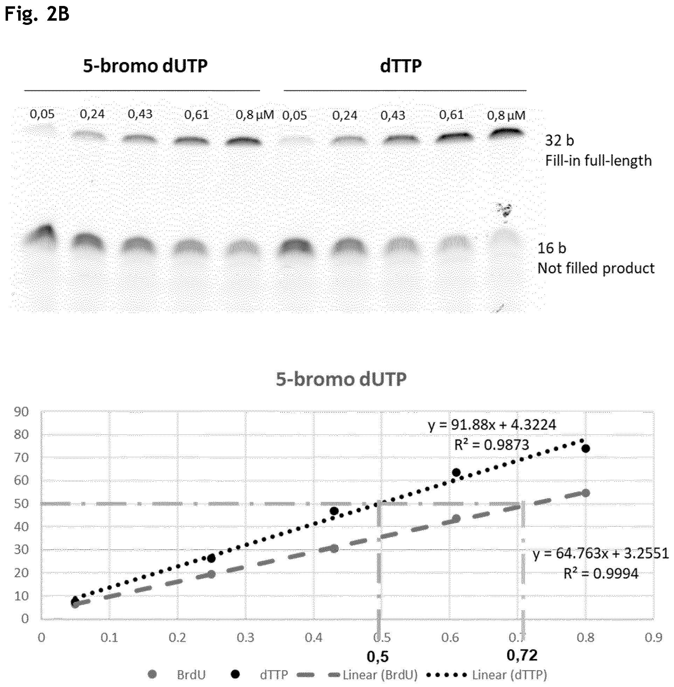

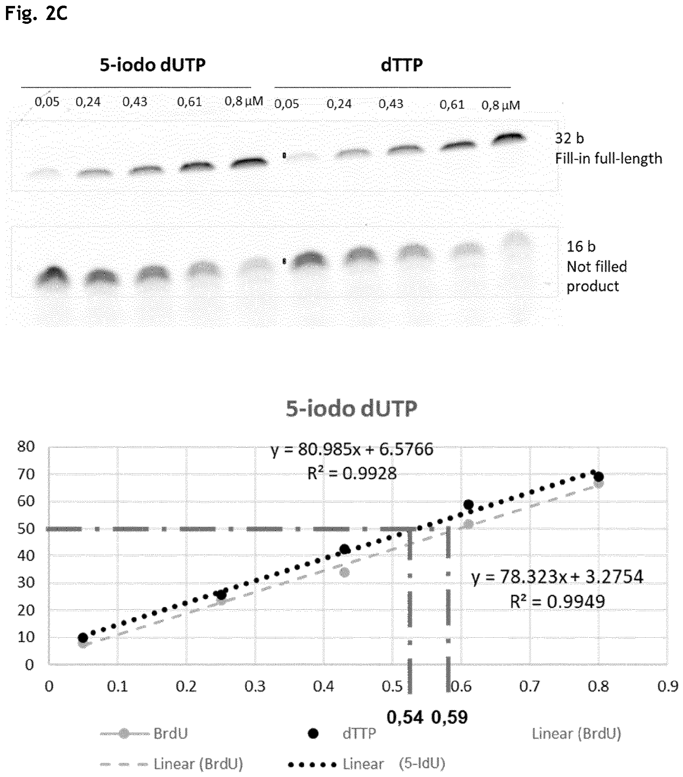

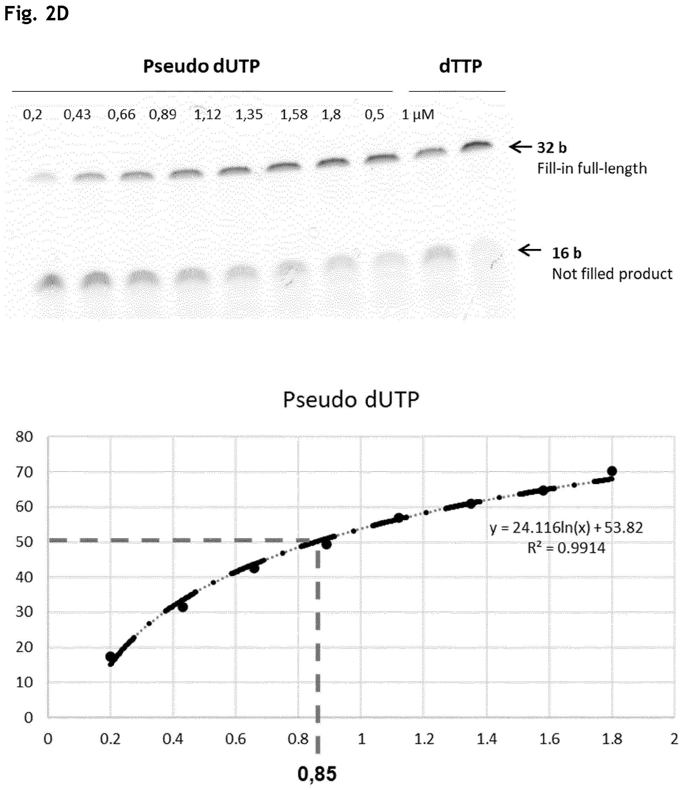

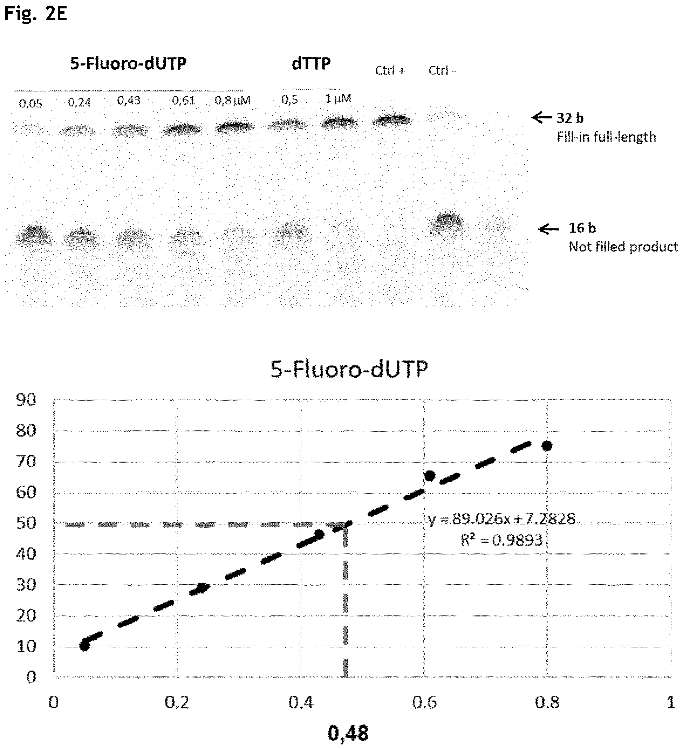

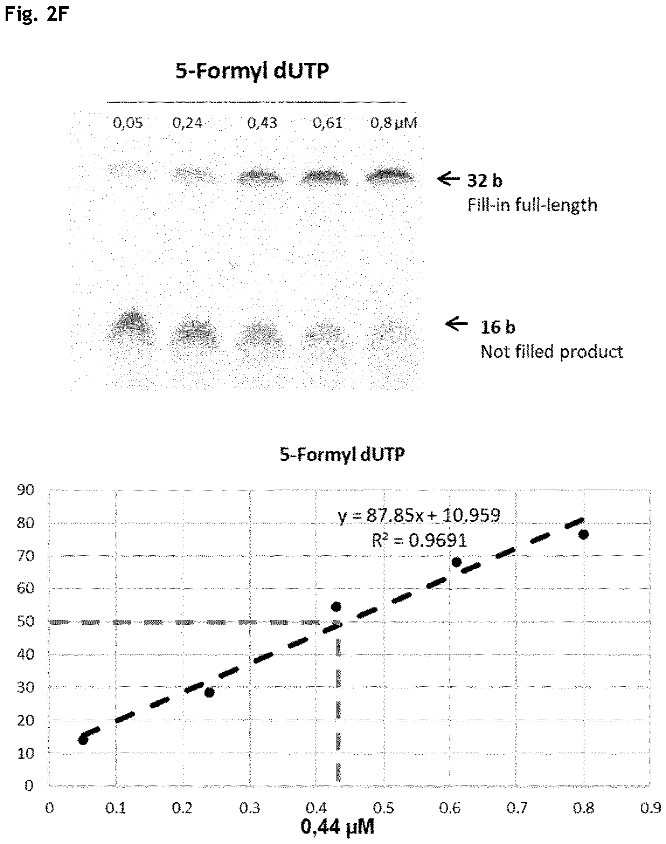

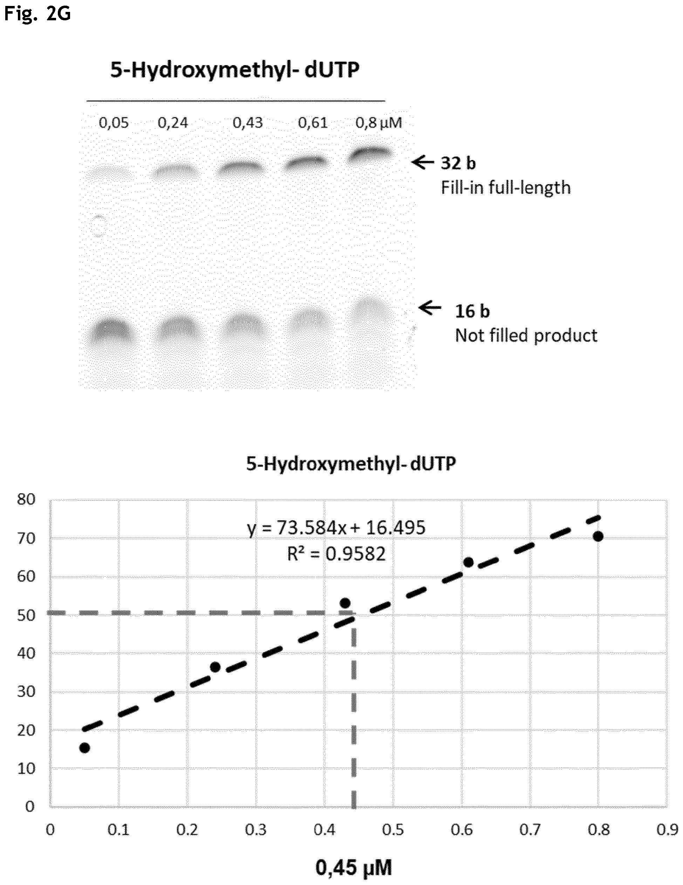

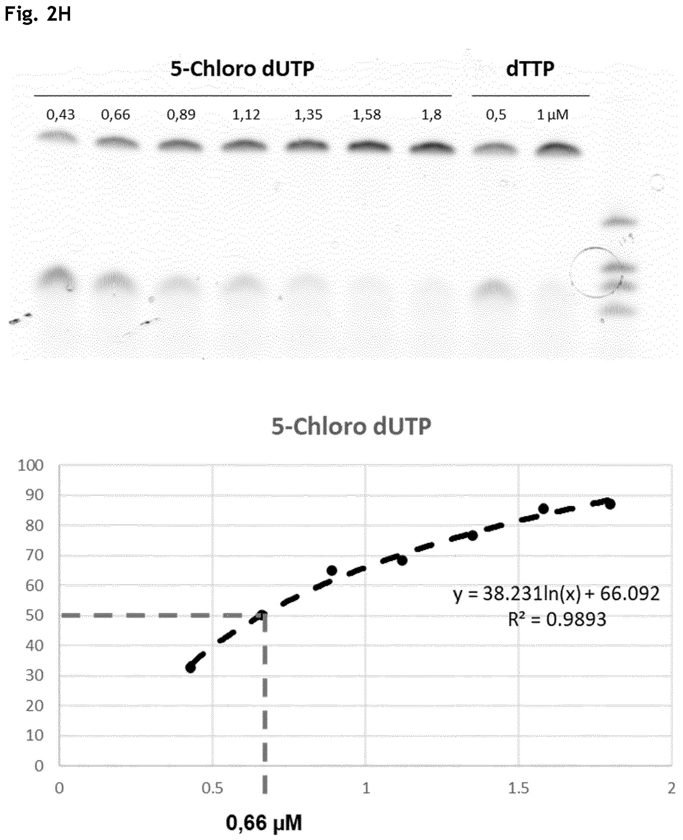

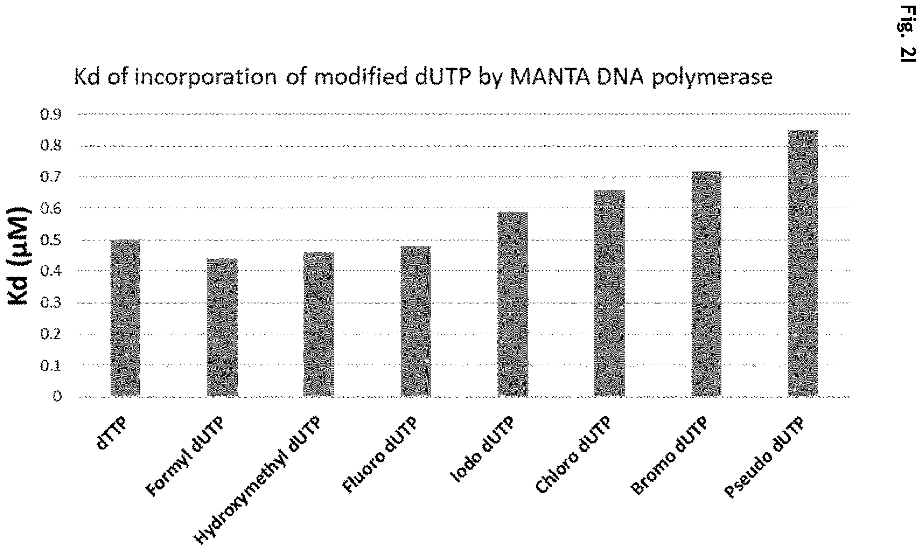

**Figure 2****: Measurement of the K_{D} of different modified uridine triphosphate by the MANTA polymerase.**
   **(A)** A template, containing only one position where a uridine can be inserted, was designed such that the 3' end of the complementary oligonucleotide tagged with FITC ends at the base just before the adenine in the template. The reaction was supplemented with dATP, dCTP and dGTP as well as the uridine triphosphate tested (no dTTP was added to the reaction). So, if the polymerase integrates the modified uridine, the FITC-labelled oligonucleotide will be filled with 16 bases, resulting in a 32 base-long labelled oligonucleotide. If the modified nucleotide is not integrated by the polymerase, the size of the FITC oligonucleotide will remain 16 bases. These two reactions product can be easily separated on a PAGE gel. (**B**) Reactions contains various concentrations of 5-bromo-dUTP as well as dTTP as positive control as indicated above each well on the gel picture were run on a PAGE gel (left panel). The ratio of fully extended product over the total amount of FITC-tagged oligonucleotide was calculated and plotted on a graph as a function of the concentration. A simple first order equation was derived from these points and the K_{D} was calculated as the 50% of the reaction kinetics (right panel). (**C**) Reactions contains various concentrations of 5-iodo-dUTP as well as dTTP as positive control as indicated above each well on the gel picture were run on a PAGE gel (left panel). The ratio of fully extended product over the total amount of FITC-tagged oligonucleotide was calculated and plotted on a graph as a function of the concentration. A simple first order equation was derived from these points and the K_{D} was calculated as the 50% of the reaction kinetics (right panel). (**D**) Reactions contains various concentrations of pseudo-dUTP as indicated above each well on the gel picture were run on a PAGE gel (left panel). The ratio of fully extended product over the total amount of FITC-tagged oligonucleotide was calculated and plotted on a graph as a function of the concentration. The best fit was obtained by using a non-linear equation from these points and the K_{D} was calculated as the 50% of the reaction kinetics (right panel). (**E**) Reactions contains various concentrations of 5-fluoro-dUTP as indicated above each well on the gel picture were run on a PAGE gel (left panel). The ratio of fully extended product over the total amount of FITC-tagged oligonucleotide was calculated and plotted on a graph as a function of the concentration. A simple first order equation was derived from these points and the K_{D} was calculated as the 50% of the reaction kinetics (right panel). (**F**) Reactions contains various concentrations of 5-formyl-dUTP as indicated above each well on the gel picture were run on a PAGE gel (left panel). The ratio of fully extended product over the total amount of FITC-tagged oligonucleotide was calculated and plotted on a graph as a function of the concentration. A simple first order equation was derived from these points and the K_{D} was calculated as the 50% of the reaction kinetics (right panel). (**G**) Reactions contains various concentrations of 5-hydroxymethyl-dUTP as indicated above each well on the gel picture were run on a PAGE gel (left panel). The ratio of fully extended product over the total amount of FITC-tagged oligonucleotide was calculated and plotted on a graph as a function of the concentration. A simple first order equation was derived from these points and the K_{D} was calculated as the 50% of the reaction kinetics (right panel). (**H**) Reactions contains various concentrations of 5-chloro-dUTP as indicated above each well on the gel picture were run on a PAGE gel (left panel). The ratio of fully extended product over the total amount of FITC-tagged oligonucleotide was calculated and plotted on a graph as a function of the concentration. The best fit was obtained by using a non-linear equation from these points and the K_{D} was calculated as the 50% of the reaction kinetics (right panel). (**I**) Summary of all the K_{D} calculated for the different modified nucleotides. The majority of the modified nucleotides have a K_{D} similar to dTTP, except for pseudouridine which is almost twice the K_{D} of dTTP.

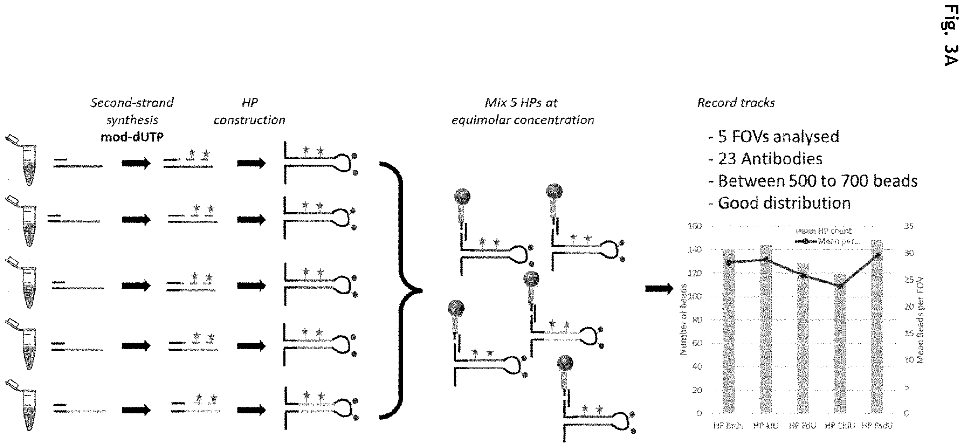

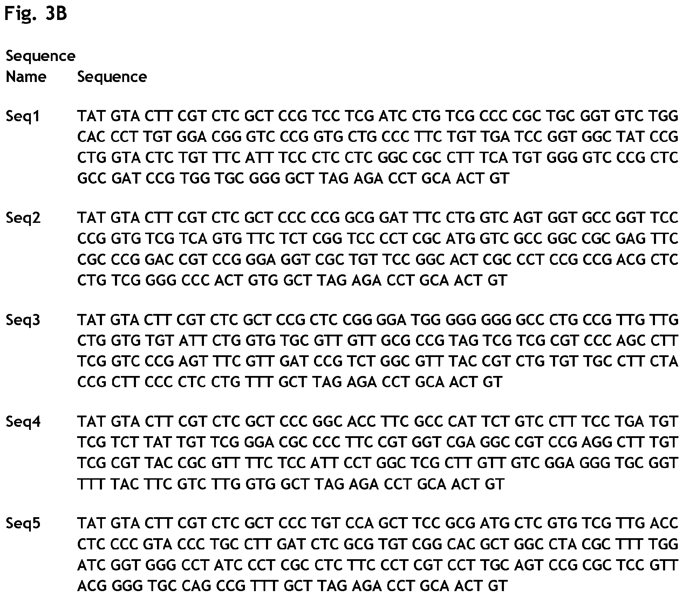

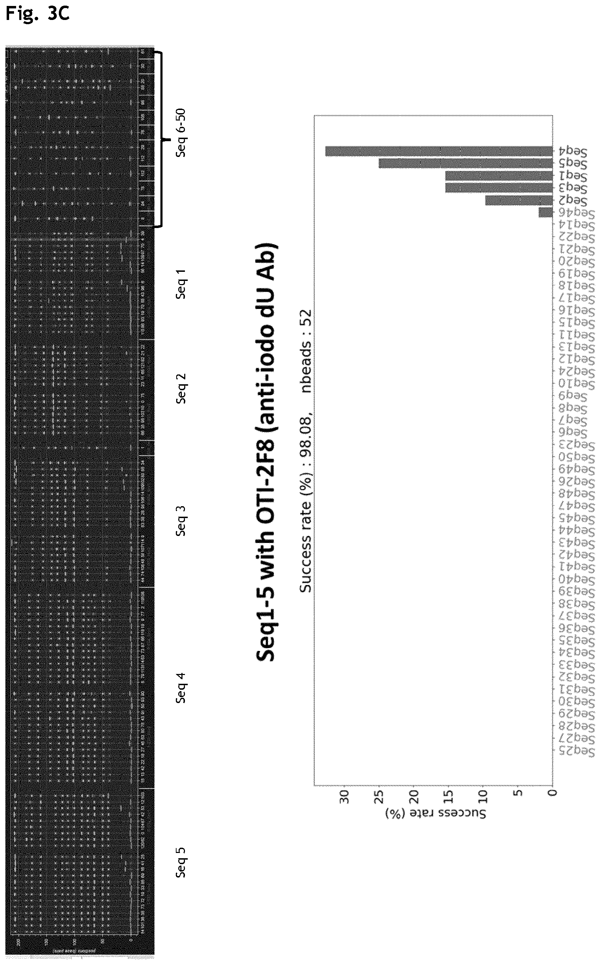

**Figure 3****: Generation of fragments with different signatures and their identification using antibody-specific signatures.**
   (**A**) Illustration of the strategy used to construct the hairpin molecules. Fifty different sequences with 10-12 positions where a modified uridine can be inserted were generated. A polymerisation reaction was then performed with the Manta polymerase along with the modified iodo-dUTP at 100% (to replace the natural dTTP). The adapters (loop and Y-shape) were then ligated to the fragments to generate the hairpin. (**B**) The first five oligonucleotides (1 to 5) were mixed in equimolar concentration. The polymerisation reaction was performed as described in (A) to generate a mix of sequences. (**C**) The hairpin molecules produced in (B) were attached to the surface of the flow cell. The antibody against the IdU modification (Anti-IdU antibody, clone OTI2F8 from Abcam) was then injected. The experimental blockages due to the antibody were extracted and fitted to the original 50 sequences to determine the identity of the molecule. It was observed that >98% of the molecules observed experimentally could be assigned to the sequence 1 to 5 from the original mix (N = 52 beads).

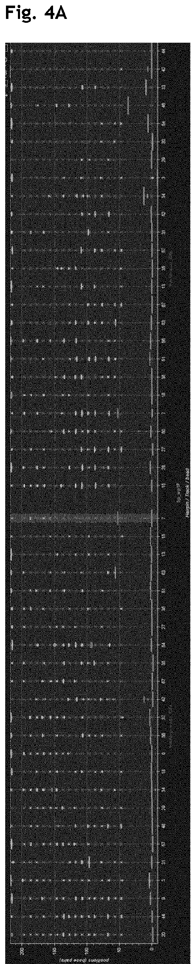

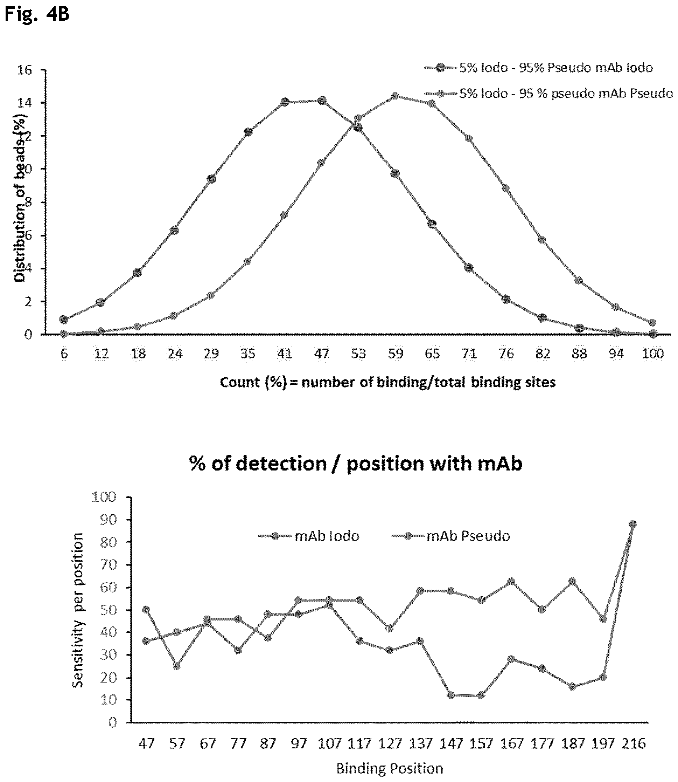

**Figure 4****: Generation of molecules with more than one modified nucleotide.**
   (**A**) One of the 50 long oligonucleotides was used as a template to incorporate two different modified nucleotides at the same time and demonstrate that it is possible to integrate more than one modified base during the polymerisation step. Both iodo-dUTP as well as pseudo-dUTP were included in the nucleotide mix during the fill it step to completely replace the natural base dTTP and we were aiming for an equal distribution of both modifications. After synthesis, the hairpin was assembled and assessed with both anti-iodouridine antibody and anti-pseudouridine antibody. (**B**) For each molecule, we counted the number of positions detected by either the anti-IdU or anti-pseudouridine over the expected total number of positions for this sequence. We then plotted the distribution of each modification for all the functional molecules. An equal distribution of both modified bases on the template was observed, i.e., there was as much IdU as pseudoUridine detected in the final hairpin produced, confirming that it is possible to integrate multiple modified bases in the same reaction. However, since K_{D} of the polymerase is lower for IdU then pseudouridine, the concentration of pseudouridine triphosphate IdU triphosphate was adjusted to obtain this equal distribution in the final hairpin.

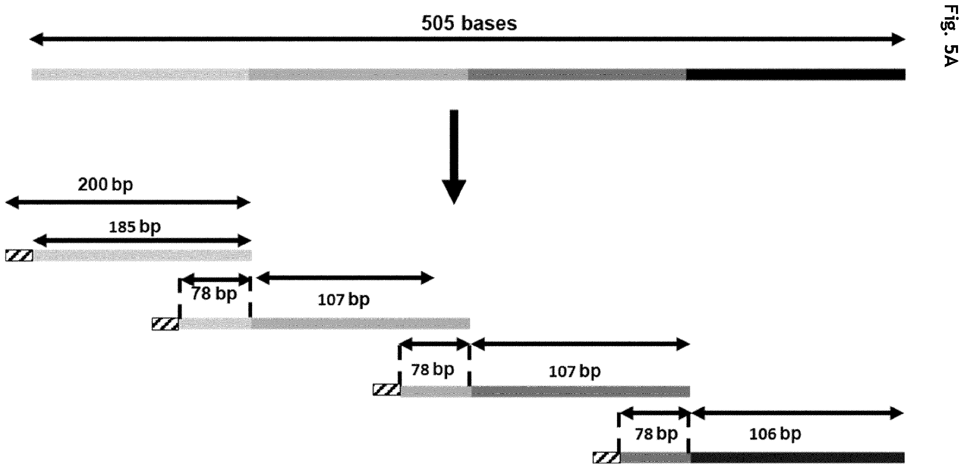

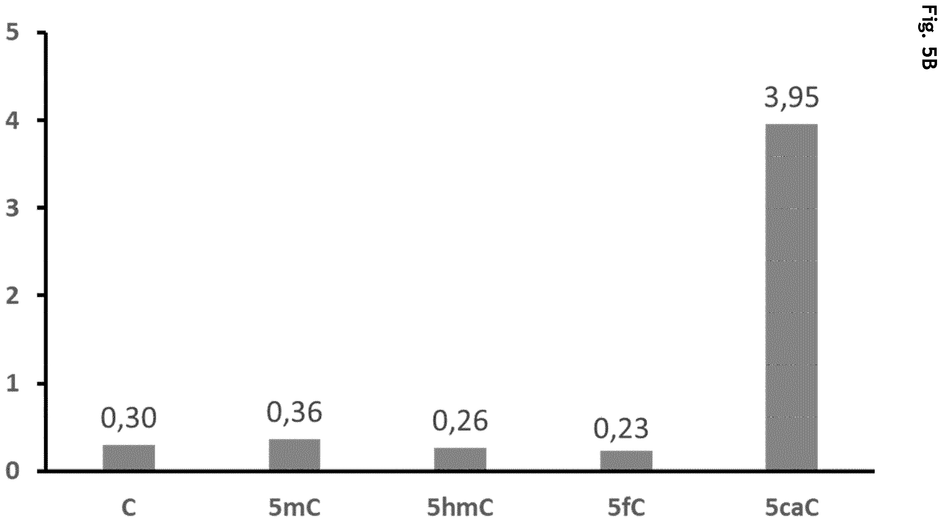

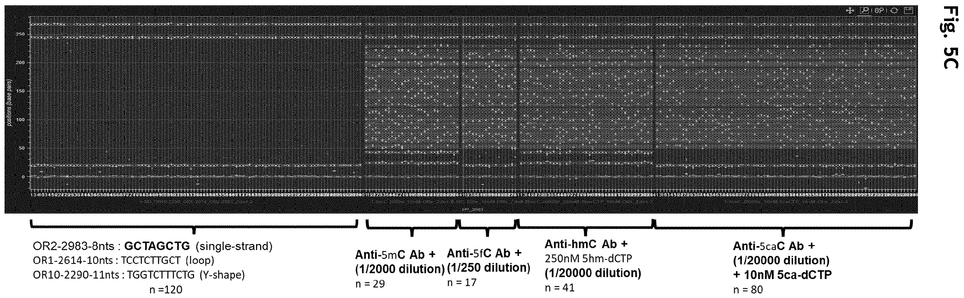

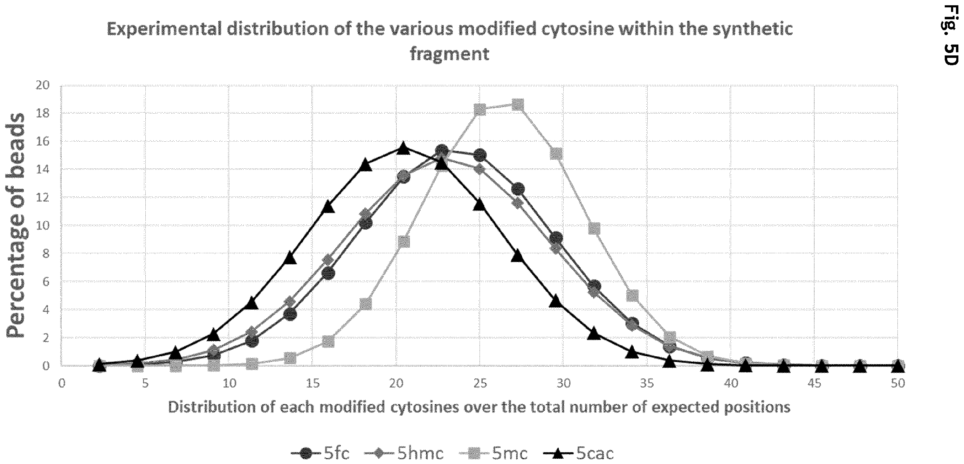

**Figure 5****: Detection of four different modified bases on the same template.**
   (**A**) As we have identified antibodies against four modified cytosines (5mC, 5hmC, 5caC and 5formylC), we used these modified bases to generate a hairpin molecule with four different modified bases in order to generate a unique signature. For this proof-of-concept, we generated a synthetic sequence of 500 base pairs where there were at least 3 bases between each site where a modified nucleotide can be inserted. We divided this 500 bases pair synthetic sequence into four long oligonucleotides in order to use them as templates to generate the hairpin molecules. Each of these oligonucleotides contain a constant sequence for construction of the hairpin (red block), a unique sequence of the 500 base sequence that about 80 bases of overlapping sequence with the previous oligonucleotide. (**B**) The K_{D} of the different modified cytosine bases we used with the MANTA DNA polymerase was determined as in Fig. 1. Except for 5caC, all the modified bases are used by the polymerase at a similar level as the natural dCTP. (**C**) The approach described in Fig. 2A was used to produce hairpin molecules. They were challenged the resulting hairpin molecules with specific antibodies against each of the four modified bases, one at the time. This figure shows the positions detected by each antibody. (**D**) As the antibodies are specific for the modifications they are intended to detect, we could estimate the distribution of each modified base on the hairpin molecules by counting the number of blockages due to one antibody over the total number of sites. As our initial mix of nucleotides contains four modified cytosines, we were aiming for an equal distribution of each of them and this is what we observed (about 25% of each modified base).

### DETAILED DESCRIPTION

The present disclosure will become more fully understood from the detailed description given herein and from the accompanying drawings, which are given by way of illustration only and do not limit the intended scope of the disclosure.

### Definitions

Unless specifically defined, all technical and scientific terms used herein have the same meaning as commonly understood by a skilled artisan in chemistry, biochemistry, cellular biology, molecular biology, and medical sciences.

The term **"about"** or **"approximately"** refers to the normal range of error for a given value or range known to the person of skills in the art. It usually means within 20%, such as within 10%, or within 5% (or 1% or less) of a given value or range.

As used herein, **"amplify", "amplifying"** or **"amplification reaction"** and their derivatives, refer generally to any action or process whereby at least a portion of a nucleic acid molecule (referred to as a template nucleic acid molecule) is replicated or copied through a template-dependent in vitro enzyme-catalysed reaction into at least one additional nucleic acid molecule. The template nucleic acid molecule can be single-stranded or double-stranded and the additional nucleic acid molecule can independently be single-stranded or double-stranded. Amplification optionally includes linear or exponential replication of a nucleic acid molecule.

The term **"antibody"** as used herein is used herein in the broadest sense and specifically covers monoclonal antibodies (including full length monoclonal antibodies) of any isotype such as IgG, IgM, IgA, IgD, and IgE, polyclonal antibodies, multispecific antibodies, chimeric antibodies, and antibody fragments, provided that the fragments retain the desired biological function. An antibody (or "immunoglobulin") consists of a glycoprotein comprising at least two heavy (H) chains and two light (L) chains interconnected by disulfide bonds. Each heavy chain comprises a heavy chain variable region (or domain) (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain, CL. The VH and VL regions can be further subdivided into regions of hypervariability, termed "complementarity determining regions" (CDR) or "hypervariable regions", which are primarily responsible for binding an epitope of an antigen, and which are interspersed with regions that are more conserved, termed framework regions (FR). Method for identifying the CDRs within light and heavy chains of an antibody and determining their sequence are well known to the skilled person. For the avoidance of doubt, in the absence of any indication in the text to the contrary, the expression CDRs means the hypervariable regions of the heavy and light chains of an antibody as defined by IMGT, wherein the IMGT unique numbering provides a standardized delimitation of the framework regions and of the complementary determining regions, CDR1-IMGT: 27 to 38, CDR2. An antibody reactive with a specific antigen can be generated by recombinant methods such as selection of libraries of recombinant antibodies in phage or similar vectors, or by immunising an animal with the antigen or an antigen-encoding nucleic acid.

The terms **"antibodies that bind to a modified nucleotide," "antibodies that bind to a nucleotide analogue",** and analogous terms are used interchangeably herein and refer to antibodies that bind to a modified nucleotide, notably those described herein. Such antibodies include polyclonal and monoclonal antibodies, including chimeric, humanised, and human antibodies. An antibody that binds to a nucleotide analogue can be identified, for example, by immunoassays, BIAcore, or other techniques known to those of skill in the art. An antibody binds to a modified nucleotide, for example, when it binds to the modified nucleotide with higher affinity than to any cross-reactive antigen (e.g., a natural nucleotide) as determined using experimental techniques, such as radioimmunoassays (RIA) and enzyme-linked immunosorbent assays (ELISAs). Typically, a specific or selective reaction will be at least twice background signal or noise and may be more than 10 times background. See, e.g., Paul, ed., 1989, Fundamental Immunology Second Edition, Raven Press, New York at pages 332-336 for a discussion regarding antibody specificity. In some embodiments, an antibody "which binds" an antigen of interest is one that binds the antigen with sufficient affinity such that the antibody is useful as a diagnostic and/or therapeutic agent in targeting a cell or tissue expressing the antigen, and does not significantly cross-react with other proteins. In such embodiments, the extent of binding of the antibody to a "non-target" protein will be less than about 10% of the binding of the antibody to its particular target protein as determined by fluorescence activated cell sorting (FACS) analysis or radioimmunoprecipitation (RIPA). With regard to the binding of an antibody to a target molecule, the term "specific binding" or "specifically binds to" or is "specific for" a particular polypeptide or an epitope on a particular polypeptide target means binding that is measurably different from a nonspecific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labelled target. In this case, specific binding is indicated if the binding of the labelled target to a probe is competitively inhibited by excess unlabelled target. The term "specific binding" or "specifically binds to" or is "specific for" a particular polypeptide or an epitope on a particular polypeptide target as used herein can be exhibited, for example, by a molecule having a K_{D} for the target of at least about 10⁻⁴ M, alternatively at least about 10⁻⁵ M, alternatively at least about 10⁻⁶ M, alternatively at least about 10⁻¹ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. In some embodiments, the term "specific binding" refers to binding where a molecule binds to a particular polypeptide or epitope on a particular polypeptide without substantially binding to any other polypeptide or polypeptide epitope. In some embodiments, an antibody that binds to the modified nucleotide has a dissociation constant (K_{D}) of ≤ 1µM, ≤ 100 nM, ≤ 10 nM, ≤ 1nM, or ≤ 0.1nM.

The term **"antigen binding fragment", "antigen binding domain", "antigen binding region",** and similar terms refer to that portion of an antibody which comprises the amino acid residues that interact with an antigen and confer on the binding agent its specificity and affinity for the antigen (e.g., the complementarity determining regions (CDRs)). By the expression "antigen-binding fragment" of an antibody, it is intended to indicate any peptide, polypeptide, or protein retaining the ability to bind to the target (also generally referred to as antigen) of the antibody, generally the same epitope, and comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, or at least 200 contiguous amino acid residues, of the amino acid sequence of the antibody. In a particular embodiment, the antigen-binding fragment comprises at least one CDR of the antibody from which it is derived. Still in a preferred embodiment, the antigen binding fragment comprises 2, 3, 4 or 5 CDRs, more preferably the 6 CDRs of the antibody from which it is derived.

The "antigen-binding fragments" can be selected, without limitation, in the group consisting of Fab, Fab', (Fab')₂, Fv, scFv (sc for single chain), Bis-scFv, scFv-Fc fragments, Fab2, Fab3, minibodies, diabodies, triabodies, tetrabodies, and nanobodies, and fusion proteins with disordered peptides such as XTEN (extended recombinant polypeptide) or PAS motifs, and any fragment of which the half-life time would be increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation in a liposome, the fragments having at least one of the characteristic CDRs of the antibody according to the invention. Among the antibody fragments, Fab has a structure including variable regions of light chain and heavy chain, a constant region of a light chain, and the first constant region of a heavy chain (CH1), and it has one antigen binding site. Fab' is different from Fab in that it has a hinge region including one or more cysteine residues at C terminus of heavy chain CH1 domain. F(ab')2 antibody is generated as the cysteine residues of the hinge region of Fab' form a disulfide bond. Fv is a minimum antibody fragment which has only a heavy chain variable region and a light chain variable region, and a recombination technique for producing the Fv fragment is described in International Publication WO 88/10649 or the like. In double chain Fv (dsFv), the heavy chain variable region and light chain variable region are linked to each other via a disulfide bond, and, in single chain Fv (scFv), the heavy chain variable region and light chain variable region are covalently linked to each other via a peptide linker in general. Those antibody fragments can be obtained by using a proteinase (e.g., Fab can be obtained by restriction digestion of whole antibody with papain or ficin, and F(ab')2 fragment can be obtained by restriction digestion with pepsin), and it can be preferably produced by genetic engineering techniques. Preferably, the "antigen-binding fragments" will be constituted or will comprise a partial sequence of the heavy or light variable chain of the antibody from which they are derived, the partial sequence being sufficient to retain the same specificity of binding as the antibody from which it is descended and a sufficient affinity, preferably at least equal to 1/100, in a more preferred manner to at least 1/10, of the affinity of the antibody from which it is descended, with respect to the target. Such antibody fragments can be found described in, for example, Harlow and Lane, Antibodies: A Laboratory Manual, Cold Spring Harbor Laboratory, New York (1989); Myers (ed.), Molec. Biology and Biotechnology: A Comprehensive Desk Reference, New York: VCH Publisher, Inc.; Huston et al., Cell Biophysics, 22:189-224 (1993); Plückthun and Skerra, Meth. Enzymol., 178:497-515 (1989) and in Day, E.D., Advanced Immunochemistry, Second Ed., Wiley-Liss, Inc., New York, NY (1990).

As used herein, **"base pair"** or **"bp"** refers to a partnership of adenine (A) with thymine (T) or uracil (U), or of cytosine (C) with guanine (G) in a double stranded nucleic acid molecule. Also included within this definition are partnerships involving an analogue of the one of these natural bases.

The terms **"binds"** or **"binding"** as used herein refer to an interaction between molecules to form a complex which, under physiologic conditions, is relatively stable. Interactions can be, for example, non-covalent interactions including hydrogen bonds, ionic bonds, hydrophobic interactions, and/or van der Waals interactions. A complex can also include the binding of two or more molecules held together by covalent or non-covalent bonds, interactions or forces. Such interactions are generally characterised by a dissociation constant (K_{D}) of 10⁻⁶ M⁻¹ or lower. **"Affinity"** refers to the strength of binding: increased binding affinity being correlated with a lower K_{D}. In particular, the strength of the total non-covalent interactions between a single antigen-binding site on an antibody and a single epitope of a target molecule, such as a modified nucleotide, is the affinity of the antibody or functional fragment for that epitope. The ratio of association (*kₒₙ*) to dissociation (*k_{off}*) of an antibody to a monovalent antigen (*kₒₙ*/ *k_{off}*) is the association constant K_{D}, which is a measure of affinity. The value of K_{D} varies for different complexes of antibody and antigen and depends on both *kₒₙ* and *k_{off}.* The association constant K_{D} for an antibody provided herein can be determined using any method provided herein or any other method well known to those skilled in the art. The affinity at one binding site does not always reflect the true strength of the interaction between an antibody and an antigen.

A **"biological sample"** as used herein refers to a sample which may be obtained from a biological organism, such as a cellular extract obtained from bacteria, viruses, plants, yeasts etc. A "biological sample" as used herein refers notably to a whole organism or a subset of its tissues, cells or component parts (e.g., blood vessel, including artery, vein and capillary, body fluids, including but not limited to blood, serum, mucus, lymphatic fluid, synovial fluid, cerebrospinal fluid, saliva, amniotic fluid, amniotic cord blood, urine, vaginal fluid and semen). "Biological sample" further refers to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as proteins or nucleic acid molecules. A "biological sample" must allow for the identification of small nucleic acid molecules. Preferred biological samples for the quantification of genomic sequences include body fluids, including but not limited to blood, plasma, serum, mucus, lymphatic fluid, synovial fluid, cerebrospinal fluid, saliva, amniotic fluid, amniotic cord blood, urine, vaginal fluid and semen, preferably blood, serum and plasma. Preferably, the biological sample is a sample of blood, plasma, or serum; more preferably, blood. Indeed, such a blood sample may be obtained by a completely harmless blood collection from a pregnant mother and thus allows for e.g., a non-invasive diagnosis of foetal aneuploidy. Also, a blood sample can be used from a cancer patient and, for example, used to characterise a liquid tumour by the method disclosed herein.

The terms **"cell proliferative disorder"** and **"proliferative disorder"** refer to disorders that are associated with some degree of abnormal cell proliferation. In some embodiments, the cell proliferative disorder is a tumour or cancer. "Tumour", as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumour" are not mutually exclusive as referred to herein. The terms "cancer" and "cancerous" refer to or describe the physiological condition in mammals that is typically characterised by unregulated cell growth. Examples of cancer include, but are not limited to, carcinoma, lymphoma, blastoma, sarcoma, and leukaemia or lymphoid malignancies. More particular examples of such cancers include squamous cell cancer (*e.g*. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, oral cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain cancer, as well as head and neck cancer, and associated metastases. In some embodiments, the cancer is a haematological cancer, which refers to cancer that begins in blood-forming tissue, such as the bone marrow, or in the cells of the immune system. Examples of a hematologic cancer are leukaemia (*e.g.*, acute myeloid leukaemia (AML), acute lymphoblastic leukaemia (ALL), chronic myelogenous leukaemia (CML), chronic lymphocytic leukaemia (CLL), or acute monocytic leukaemia (AMoL)), lymphoma (Hodgkin lymphoma or non-Hodgkin lymphoma), and myeloma (multiple myeloma, plasmacytoma, localised myeloma or extramedullary myeloma).

The term **"circulating nucleic acid"** or **"CNA**" as used herein refers to genomic, mitochondrial or viral DNA, RNA (including mRNA) and small RNAs (including microRNA (miRNA), as well as segments or portions thereof, found in a biological fluid of a subject. Circulating DNA and RNA are commonly known as **"cell-free DNA"** or **"cfDNA"** and **"cell-free RNA"** or **"cfRNA".** CNAs may be found in particular in the bloodstream (including blood, plasma, serum, lymph), as well as in other biological fluids (such as urine). Methods of detecting and/or isolating CNAs are well known by the person skilled in the art and include, for example, any methods for detecting and/or isolating nucleic acids from a biological fluid (examples of such methods as described hereinafter). Cells present in the biological fluid are preferably removed (e.g., by techniques well known by the skilled person, such as filtering, centrifugating, etc.) to avoid the presence of contaminating cellular nucleic acid. For example, any commercial kits designed for isolating circulating DNA from plasma may be used (such as the QIAamp^{®} Circulating Nucleic Acid kit (Qiagen^{®}), the Maxwell^{®} Rapid Sample Concentrator (RSC) ccfDNA Plasma Kit (Maxwell^{®}), the Zymo^{®} Quick ccfDNA Serum & Plasma Kit (Zymo Research^{®}), the QIAamp^{®} MinElute ccfDNA Midi Kit^{™} (Qiagen^{®}), Norgen^{®} Plasma/Serum RNA/DNA Purification Mini Kit (Norgen Biotek^{®}), and the like).

As used herein, the terms **"complementary"** or **"complementarity",** when used in reference to nucleic acids (i.e., a sequence of nucleotides such as an oligonucleotide or a target nucleic acid), refer to sequences that are related by base-pairing rules. For natural bases, the base pairing rules are those developed by Watson and Crick. For modified bases, as described herein, the base-pairing rules include the formation of hydrogen bonds in a manner similar to the Watson-Crick base pairing rules or by hydrophobic, entropic, steric or van der Waals forces.

As used herein, the term **"denaturation"** refers to the process of strands separation of a double-stranded nucleic acid molecule occurring when most of the hydrogen bonds between the strands are broken. The denaturation process yields a denatured nucleic acid molecule, by which it is herein meant the two separated complementary strands resulting from the denaturation of a double-stranded nucleic acid molecule. The denaturation may be partial, i.e., some of the hydrogen bonds between the two strands remain intact, or total, wherein all of the hydrogen bonds are broken.

The term **"detecting"** as used herein encompasses quantitative or qualitative detection.

As used herein, **"diagnosis"** or **"identifying a subject having"** refers to a process of identifying a disease, condition, or injury from its signs and symptoms. A diagnosis is notably a process of determining if an individual is afflicted with a disease or ailment (e.g., cancer). Cancer is diagnosed for example by detecting either the presence of a marker associated with cancer.

By **"epigenetic modifications",** it is herein referred to modifications of the bases constituting a nucleic acid molecule which take place after the synthesis of the nucleic acid molecule. Such epigenetic modifications include, inter alia, 4-methylcytosine (m4C), 5-methylcytosine (5mC), 5-hydroxymethylcytosine (5hmC), 5-formylcytosine (5fC) and 5-carboxylcytosine (5caC), as well as 6-methyladenosine (m6A) in DNA, and 5-hydroxymethyluracil (5hmU) and N6-methyladenosine (m6A) in RNA (see e.g., Kumar et al. Front Genet. 9: 640, 2018, for a review).

The term **"fragment"** is intended to encompass a portion of a nucleic acid or a protein. A nucleic acid fragment may be at least about 15 contiguous nucleotides, preferably at least about 18, 20, 23 or 25 nucleotides, and can be 30, 40, 50, 100, 200 or more nucleotides in length. A protein fragment may be at least about 5 contiguous amino acids in length, preferably at least about 7, 10, 15, or 20 amino acids, and can be 25, 30, 40, 50 or more amino acids in length. The term **"antibody fragment"** refers to a portion of a full-length antibody, generally the target binding or variable region. Examples of antibody fragments include Fab, Fab', F(ab')2 and Fv fragments. An "Fv" fragment is the minimum antibody fragment which contains a complete target recognition and binding site. This region consists of a dimer of one heavy and one light chain variable domain in a tight, non-covalent association (V_{H}-V_{L} dimer). It is in this configuration that the three CDRs of each variable domain interact to define a target binding site on the surface of the V_{H}-V_{L} dimer. Often, the six CDRs confer target binding specificity to the antibody. However, in some instances even a single variable domain (or half of an Fv comprising only three CDRs specific for a target) can have the ability to recognise and bind target, although at a lower affinity than the entire binding site. "Single-chain Fv" or "scFv" antibody fragments comprise the V_{H} and V_{L} domains of an antibody, wherein these domains are present in a single polypeptide chain. Generally, the Fv polypeptide further comprises a polypeptide linker between the V_{H} and V_{L} domains which enables the scFv to form the desired structure for target binding. "Single domain antibodies" are composed of a single V_{H} or V_{L} domains which exhibit sufficient affinity to the antigen, e.g., a modified nucleotide. In a specific embodiment, the single domain antibody is a camelised antibody (See, *e.g.*, Riechmann, 1999, Journal of Immunological Methods 231:25-38).

The Fab fragment contains the constant domain of the light chain and the first constant domain (CH₁) of the heavy chain. Fab' fragments differ from Fab fragments by the addition of a few residues at the carboxyl terminus of the heavy chain CHₗ domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

As used herein, a **"hairpin"** means a nucleic acid molecule comprising a region of intra-strand pairing linked to a loop. A **"loop",** as used herein, refers to a succession of nucleotides of a strand of the nucleic acid that are not paired through hydrogen bonds with nucleotides of the same or another strand of the nucleic acid. The region of intra-strand pairing is usually referred to as a **"stem"** and may comprise at least 3, 4, 5, 6, 7, 8, 9, 10, 15, 20, 40, or 60 pairs of bases.

As used herein, the term **"hybridisation"** refers to the process of establishing a non-covalent, sequence-specific interaction between two or more complementary strands of nucleic acids into a single hybrid.

As used herein, a **"nucleoside"** refers to a compound containing a five-carbon sugar molecule (a pentose or ribose) or derivative thereof, and an organic base, purine or pyrimidine, or a derivative thereof.

As used herein, a **"nucleotide"** is defined as a nucleoside comprising a phosphate group.

The term **"conventional"** or **"natural"** when referring to nucleic acid bases, nucleoside triphosphates, or nucleotides refers to those which occur naturally in the polynucleotide being described (i.e., for DNA these are dATP, dGTP, dCTP and dTTP). The bases incorporated in naturally-occurring DNA and RNA are adenosine (A), guanosine (G), thymidine (T), cytosine (C), and uridine (U). These five bases are **"natural bases".** According to the rules of base pairing elaborated by Watson and Crick, the natural bases can hybridise to form purine-pyrimidine base pairs, where G pairs with C and A pairs with T or U. These pairing rules facilitate specific hybridisation of a nucleic acid with a complementary nucleic acid. "Natural bases" as used herein also encompass bases which are only found in context of one or more particular nucleic acid species. For example, modified bases resulting from epigenetic modifications are natural bases. Likewise, tRNAs and rRNAs may comprise several bases not found in any other nucleic acid species, such as e.g., dihydrouridine, pseudouridine, inosine, queuosine, uridine-5-oxyacetic acid, 5-methylaminomathyl-2-thiouridine, lysidine, 2-methyladenine, *N*⁶-methyladenine, *N*⁶-dimethyladenine, 5-methylcytosine, methoxycaronyl-methyl-2-thiouridine, etc. These bases are well known to the skilled person and are described in publications such as e.g., Jonkhout et al. RNA. 2017 23(12):1754-1769; Agris et al. Enzymes. 2017 41 :1-50; and Helm et al. Chem Biol. 2014 21 (2):174-85, as well as in textbooks such as Genes VIII, Lewin B, Pearson; United States (2003).

The term **"modified nucleotide", "nucleotide analogue",** or **"modified base"** as used herein refers to nucleotides and bases which do not naturally occur in the nucleic acid molecule of interest. "Modified nucleotides" and "modified bases" as used herein thus refers to nucleotides and bases other than the nucleotides and bases which occur naturally in the nucleic acid of interest. "Modified nucleotides" and "modified bases" as used herein include nucleotides and bases which are normally found in other nucleic acid species but which are not found in the nucleic acid of interest. For example, pseudouridine is a modified base when present in DNA but is a natural base in tRNA or rRNA. As used herein, "modified nucleotide", "nucleotide analogue", or "modified base" also include non-natural nucleotides and non-natural bases, i.e., nucleotides and bases which do not exist in nature but are synthetic derivatives and analogues of natural bases. Such derivatives and analogues are discussed, for example, in Scheit, Nucleotide Analogs (John Wiley & Son, 1980), Uhlman et al., Chemical Reviews 90: 543-584, 1990, and Marceca et al. Sci Data. 2021 8(1):199. Preferably, such analogues and modified bases and nucleotides include synthetic equivalents of these compounds which are capable of mimicking the activity of the original nucleotides and bases, and include bases modified by halogenation, azotation, N-conjugation, and ring modification, sugar rings modified by halogenation, methylation, saturation, ring opening, and hydroxylation/de-hydroxylation; and phosphates, including but not limited to analogues that have altered stacking interactions; base analogues with alternative hydrogen bonding configurations (e.g., such as iso-C and iso-G and other non-standard base pairs); non-hydrogen bonding analogues (e.g., non-polar, aromatic nucleoside analogues such as 2,4-difluorotoluene); "universal" bases such as 5-nitroindole and 3-nitropyrrole; and universal purines and pyrimidines (such as "K" and "P" nucleotides, respectively). Nucleotide analogues include modified forms of deoxyribonucleotides as well as ribonucleotides.

A "nucleic acid" as used herein refers to a single- or double-stranded linear polynucleotide containing deoxyribonucleotides and/or ribonucleotides that are linked by 3'-5'-phosphodiester bonds. A "nucleic acid" refers to either DNA or RNA, single-stranded or double-stranded, and any chemical modifications thereof. By "nucleic/acid", such hybrid molecules as DNA/RNA duplexes, i.e., a molecule comprising a DNA strand and a complementary RNA strand, are also encompassed. The terms "deoxyribonucleic acid" and "DNA" as used herein mean a polymer composed of deoxyribonucleotides. The terms "ribonucleic acid" and "RNA" as used herein mean a polymer composed of ribonucleotides. Modifications include, but are not limited to, those which provide other chemical groups that incorporate additional charge, polarisability, hydrogen bonding, electrostatic interaction, and functionality to the nucleic acid. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Examples of modified nucleotides include, but are not limited to 2,6-Diaminopurine (2-Amino-dA), 5-Methyl dC, locked nucleic acid (LNA), which are nucleotides in which the ribose moiety is modified with an extra bridge connecting the 2' oxygen and 4' carbon, unlocked nucleic acids (UNAs), which are acyclic RNA analogues without a C2'-C3' bond in the ribose ring, and peptide nucleic acid (PNA), wherein the backbone is composed of repeating N-(2-aminoethyl)-glycine units linked by peptide bonds. Modifications can also include 3' and 5' modifications such as capping.

As used herein, a **"polymerase"** is an enzyme which is capable of extending a nucleic acid by incorporating nucleic acids complementary to a template nucleic acid. Enzymes having polymerase activity catalyse the formation of a bond between the 3' hydroxyl group at the growing end of a nucleic acid primer and the 5' phosphate group of a nucleotide triphosphate. These nucleotide triphosphates are usually selected from deoxyadenosine triphosphate (A), deoxythymidine triphosphate (T), deoxycytosine triphosphate (C) and deoxyguanosine triphosphate (G). However, in at least some embodiments, polymerases useful for the methods disclosed herein also may incorporate modified bases using nucleotide triphosphates of those modified bases. A "DNA polymerase" as used herein catalyses the polymerisation of deoxynucleotides, whilst an "RNA polymerase" catalyses the polymerisation of ribonucleotides.

As used herein, the terms **"protein", "proteins", "polypeptide",** and **"polypeptides",** are synonyms and refer to polymers of amino acids covalently linked through peptide bonds into a chain. Peptide bonds are formed between the carboxyl group of one amino acid and the amino group of the next amino acid. The terms also apply to amino acid polymers in which one or more amino acids are chemical analogues or modified derivatives of corresponding naturally-occurring amino acids. The terms "amino acids" and "amino acid" refer to all naturally occurring alpha amino acids in both their D and L stereoisomeric forms, and their analogues and derivatives. An analogue is defined as a substitution of an atom in the amino acid with a different atom that usually has similar properties. A derivative is defined as an amino acid that has another molecule or atom attached to it. Derivatives would include, for example, acetylation of an amino group, amination of a carboxyl group, or oxidation of the sulfur residues of two cysteine molecules to form cystine.

Proteins can have several functions. A '**binding protein**' is a protein which is capable of binding non-covalently to another molecule. A binding protein can bind to, for example, a nucleotide (a nucleotide-binding protein), a DNA molecule (a DNA-binding protein), an RNA molecule (an RNA-binding protein) and/or a protein molecule (a protein-binding protein). In the case of a protein-binding protein, it can bind to itself (to form multimers) and/or it can bind to one or more molecules of a different protein or proteins. A binding protein can have more than one type of binding activity. For example, zinc finger proteins have DNA-binding, RNA-binding and protein-binding activity. A 'nucleic acid-binding protein' according to the invention is thus a protein which is capable of interacting with a nucleic acid. A 'single-stranded nucleic acid-binding protein' according to the invention is thus a protein which is capable of interacting with a single-stranded nucleic acid, while a 'double-stranded nucleic acid-binding protein' according to the invention is thus a protein which is capable of interacting with a double-stranded nucleic acid. A "nucleotide-binding protein" is a protein capable of interacting specifically with a nucleotide, e.g., a modified nucleotide such as those used in the present methods.

As used herein, the term **"renaturation"** refers to the process by which two separated complementary strands (single-stranded DNA and/or RNA) reform through hybridisation into a double helix. The renaturation process yields a renatured nucleic acid molecule, by which it is herein meant the double-stranded molecule resulting from the hybridisation of two complementary strands. Renatured nucleic acid molecules thus formed include DNA/DNA, RNA/RNA, and DNA/RNA duplexes. The renaturation may be partial, i.e., some of the hydrogen bonds between the two strands remain broken, or total, wherein all of the hydrogen bonds are intact.

### Identification of short nucleic acids

The present disclosure provides a method for identifying short nucleic acids, e.g., nucleic acids of less than 500bp, preferably less than 200 bp. This method relies on the use of modified nucleotides. Since they are not present naturally in the nucleic acid molecule of interest, they can be specifically distinguished from natural nucleotides. According to the method disclosed herein, at least one modified nucleotide is incorporated during replication of the short nucleic acid molecule, thus yielding molecules composed of a newly synthesised strand containing only the modified nucleotide and a native template strand. Since the modified nucleotide is a nucleotide which is not naturally present in nucleic acids, the position of the modified nucleotide in the newly-synthesised strand can be univocally identified, thereby generating a unique signature which can be used to identify the nucleic acid molecule.

This method is particularly advantageous since it only requires a minimal number of reagents to create a signature allowing identifying the fragments. Notably, the method allows single-molecule identification of the fragments. Indeed, the present method allows to detect all the positions where a modified base has been inserted within a synthesised second strand. This provides a unique signature, which can be further refined by mapping the positions of the complementary, native base within the first, native strand.

In addition, the present method allows not only to identify the specific nucleic acid fragments, but also to detect native epigenetic modifications in these fragments. This is because the modified nucleotides are not naturally found in the nucleic acid molecules of interest and therefore their detection does not interfere with the detection of natural epigenetic modifications.

In a first aspect, the method disclosed herein comprises the steps of:
a) contacting a nucleic acid molecule with a polymerase and a mix of nucleotides triphosphates, wherein at least one of the nucleotides triphosphates is a modified nucleotide triphosphate,
b) performing a replication reaction with the polymerase and the nucleotides triphosphates mix of step a), and with the nucleic acid molecule as template,
c) detecting the position of the modified nucleotide in the nucleic acid molecules obtained by the reaction of step b), thereby generating a unique signature, and
d) identifying the nucleic acid molecule based on the unique signature of step c).

The method disclosed herein can be used to identify any type of nucleic acid fragment. For example, the nucleic acid fragment may be a fragment of DNA. In another instance, the nucleic acid fragment is a fragment of RNA. Preferably, the nucleic acid fragment is double-stranded, so that replication of the fragment yields two double-stranded molecules, each comprising one strand containing the modified nucleotide and the other comprising only natural nucleotides. However, the nucleic acid fragment may also be a single-stranded, e.g., a single-stranded RNA molecule. Replication of that molecule yields one molecule comprising one strand containing the modified nucleotide and the other comprising only natural nucleotides.

As will easily be understood by the skilled person, the sensitivity of the method disclosed herein can be further improved by obtaining information from the native strand. This strand does not contain any nucleotide analogue. Yet, the position of the nucleotide analogue in the replicated strand indicates the position of the complementary base in this native strand. It is therefore advantageous to detect the position of this complementary nucleotide in the native strand. Indeed, identifying the position of this complementary nucleotide in the native strand reduces the error, thus improving the reliability of the signature. For example, by substituting dTTP (deoxythymidine triphosphate) using modified deoxyuracil triphosphates (dUTP), the polymerase will incorporate dUTP at each position where there is an adenine in the template strand. Detection of dUTPs in the replicated strand generates a signature which is unique to the nucleic acid molecule of interest and whose reliability can be further detecting by sequencing all the As on the native strand.

The position of the modified nucleotides in the newly-replicated molecules can be detected accurately because they are not found otherwise in nucleic acid molecules. Any nucleotide analogue can be used in the method of the disclosure, provided it is not present naturally in nucleic acids and can be taken up by a polymerase. Indeed, it is because these nucleotides analogues do not exist naturally in the nucleic acid molecule of interest that they can be detected unambiguously and therefore create a unique signature for the nucleic acid molecule of interest. Preferably, the modified nucleotides used in the present method are not naturally present in nucleic acids such as DNA or RNA, such that their detection will not interfere with the detection of natural epigenetic modifications.

In a first aspect, the nucleotides mix comprises one modified nucleotide. Advantageously, the mix comprises more than one modified nucleotide. This increases the density of information obtained from the nucleic acid molecule of interest. For example, the mix may comprise two or three modified nucleotides. Preferably, all four nucleotides in the mix are modified nucleotides.

Alternatively, it is also possible to reduce the density of modified bases within the replicated strand by using more than one modified base of the same nucleotide (for example, multiple dUTP-modified bases). For instance, it is possible to use 2, 3, or even 4 modified bases of the same nucleotide (for example, 5-bromo-2'-deoxyuridine-5'-triphosphate, 5-iodo-2'-deoxyuridine-5'-triphosphate, 5-fluoro-2'-deoxyuridine-5'-triphosphate, 5-ethynyl-2'-deoxyuridine-5'-triphosphate, 5-hydroxymethyl-2'-deoxyuridine-5'-triphosphate, and 5-chloro-2'-deoxyuridine-5'-triphosphate). Consequently, 50 %, 33 %, or 25 % of the inserted bases will be modified by a specific chemical group, thus improving the specific detection of each modified base.

Preferably, the modified nucleotide comprises such bases as e.g., 3-nitropyrrole 2'-deoxynucloside and 5-nitroindole 2'-deoxynucleoside, alpha phosphorothiolate, phosphorothioate nucleotide triphosphates, pyrazolo[3,4-d]pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyl uracil and cytosine, 6-azo uracil, cytosine and thymine, 5-uracil (pseudouracil), 4-thiouracil, 8-halo (e.g., 8-bromo), 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, deazaguanine, 7-deazaguanine, 3-deazaguanine, deazaadenine, 7-deazaadenine, 3-deazaadenine, pyrazolo[3,4-d]pyrimidine, imidazo[1,5-a]1,3,5 triazinones, 9-deazapurines, imidazo[4,5-d]pyrazines, thiazolo[4,5-d]pyrimidines, pyrazin-2-ones, 1,2,4-triazine, pyridazine; and 1,3,5 triazine; the modified nucleotide being preferably selected in the group consisting of 5-bromo-2'-deoxyuridine-5'-triphosphate, 5-iodo-2'-deoxyuridine-5'-triphosphate, 5-fluoro-2'-deoxyuridine-5'-triphosphate, 5-ethynyl-2'-deoxyuridine-5'-triphosphate, 5-chloro-2'-deoxyuridine-5'-triphosphate, 5-methyluridine triphosphate, 5-hydroxymethyluridine-5'-triphosphate, 1-methyl-pseudouridine-5-triphosphate, 1-methyl-pseudouridine-phosphoramidite, pseudouridine-5'-triphosphate, 5-formyl-2'-deoxyuridine-5'-triphosphate, 5-carboxy-2'-deoxyuridine-5'-triphosphate, 5-fluorouridine-5'-triphosphate; 5-methyluridine (m5U), 5-taurinomethyluridine (τm5U), 5-carboxymethylaminomethyluridine (cmnm5U), 5-hydroxymethyl-2'-deoxyuridine-5'-triphosphate, 5-hydroxyuridine (ho5U), 5-methoxyuridine (mo5U), 5-carboxymethyluridine (cmo5U), 5-carboxymethoxyuridine (mcmo5U), 5-bromo-2'-deoxycytidine-5' triphosphate, 5 iodo 2' deoxycytidine 5' triphosphate, 5 fluoro 2' deoxycytidine 5' triphosphate.

More preferably, the modified nucleotide comprises a base selected from the group consisting of 5-bromo-2'-deoxyuridine-5'-triphosphate, 5-iodo-2'-deoxyuridine-5'-triphosphate, 5-fluoro-2'-deoxyuridine-5'-triphosphate, pseudouridine-5-triphosphate, 5-formyl-2'-deoxyuridine-5'-triphosphate, 5-carboxy-2'-deoxyuridine-5'-triphosphate, 5-hydroxymethyl-2'-deoxyuridine-5'-triphosphate, and 5-chloro-2'-deoxyuridine-5'-triphosphate. Even more preferably, the base of the modified nucleotide is selected from the group consisting of 5-iodo-2'-deoxyuridine-5'-triphosphate, 5-fluoro-2'-deoxyuridine-5'-triphosphate, 5-hydroxymethyl-2'-deoxyuridine-5'-triphosphate and pseudouridine-5'-triphosphate.

Even though the methods disclosed herein can be used to identify nucleic acid molecules of any length, it is particularly advantageous to use them for identifying short nucleic acid molecules. Preferably, the nucleic acid molecule is 500 bp or less, 400 bp or less, 300 bp or less, more preferably 200 bp or less.

The nucleic acid may be circular or linear. The skilled person will easily realise that any nucleic acid type will be efficiently replicated in the conditions described above. For example, a nicked double-stranded nucleic acid molecule has a 3'-OH extremity which can be extended by the polymerase of the method disclosed herein. Alternatively, linkers can be added at the ends of a linearised nucleic acid molecule and hybridised with a primer for initiating the synthesis reaction. Methods known in the art of recombinant DNA technology may be routinely applied to this effect, and such methods are described, for example, in Ausubel et al., eds., Current Protocols in Molecular Biology, John Wiley & Sons (1993).

The polymerase used in the method may be any type of nucleic acid polymerase, as long as it can use modified nucleotides in the polymerisation reaction. Polymerases according to the invention include RNA-dependent RNA polymerases, DNA-dependent RNA polymerases, DNA-dependent DNA polymerases, RNA-dependent DNA polymerases (reverse transcriptase) and the like. Preferably, the polymerase is a DNA polymerase or an RNA dependent RNA polymerase. More preferably, the polymerase is a DNA polymerase. Numerous DNA polymerases are known in the art (e.g., T4 DNA polymerase, DNA polymerase I, Klenow Fragment, Φ29 DNA polymerase, T7 DNA polymerase, etc.). The DNA polymerase may comprise 3' to 5' exonuclease activity. The DNA polymerase may comprise 5' to 3' exonuclease activity. The DNA polymerase may comprise both 3' to 5' exonuclease activity and 5' to 3' exonuclease activity. The DNA polymerase may comprise neither 3' to 5' exonuclease activity nor 5' to 3' exonuclease activity. The DNA polymerase may comprise strand displacement activity. In some cases, the DNA polymerase does not comprise strand displacement activity. Alternatively, the polymerase descried herein is an RNA-dependent RNA polymerase. Preferably the RNA-dependent RNA polymerase is a viral RNA-dependent RNA polymerase or a eukaryotic RNA-dependent RNA polymerase. Any of the RNA-dependent RNA polymerases known to the skilled person (Zong et al. Gene. 2009 447(1): 29-39. Venkataraman et al. Viruses. 2018 10(2):76) can be used in the methods of the disclosure. In particular, examples of viral RNA dependent RNA polymerases suitable to the invention include RNA dependent RNA polymerases of polio virus, polioviral 3Dpol, vesicular stomatitis virus L, hepatitis C virus NS5B protein. See also WO2022/114767 A1, RU2783430 C1, etc.

The polymerase may be a psychrophilic, a mesophilic, or a thermophilic polymerase. Psychrophilic bacteria are defined as cold-adapted microorganisms thriving at temperatures below 15°C and tolerating 0°C. A "psychrophilic" polymerase as used herein refers to an enzyme which has an optimal temperature below 20°C, preferably, between 0°C and 20°C, more preferably between 5°C and 15°C, even more preferably between 10°C and 15°C. A "mesophilic" polymerase refers to an enzyme that has an optimum temperature of 60°C or less. Preferably, the mesophilic polymerase has an optimum temperature of 15°C to 60° C, preferably 20°C to 50°C, more preferably 25°C to 37°C. Mesophilic enzymes are in most cases not thermostable, i.e., they are irreversibly inactivated after incubation at high temperatures. In contrast, a "thermophilic" polymerase is an enzyme whose optimal temperature is of 60° C or more. Preferably, the optimal temperature of a thermophilic polymerase is comprised between 60°C and 95°C, more preferably between 65°C and 85 °C, even more preferably between 70° C and 80° C.

Most thermophilic polymerases are thermostable. The term "thermostable polymerase" refers to an enzyme that is stable to heat, is heat resistant, and retains sufficient activity to effect subsequent polynucleotide extension reactions and does not become irreversibly denatured (inactivated) when subjected to the elevated temperatures for the time necessary to effect denaturation of double-stranded nucleic acids. The heating conditions necessary for nucleic acid denaturation are well known in the art and are exemplified in, e.g., U.S. Patent Nos. 4,683,202, 4,683,195, and 4,965,188. As used herein, a thermostable polymerase is suitable for use in a temperature cycling reaction such as the polymerase chain reaction ("PCR"). Irreversible denaturation for purposes herein refers to permanent and complete loss of enzymatic activity. For a thermostable polymerase, enzymatic activity refers to the catalysis of the combination of the nucleotides in the proper manner to form polynucleotide extension products that are complementary to a template nucleic acid strand. Thermostable DNA polymerases from thermophilic bacteria include, e.g., DNA polymerases from *Thermotoga maritima, Thermus aquaticus, Thermus thermophilus, Thermus flavus, Thermus filiform is, Thermus* species sps17, *Thermus* species Z05, *Thermus caldophilus, Bacillus caldotenax, Thermotoga neopolitana,* and *Thermosipho africanus.*

The polymerase according to the disclosure may be a processive polymerase. A processive enzyme catalyses multiple rounds of a reaction on a denatured double-stranded nucleic acid template, while the enzyme stays bound to the template. As understood herein, a polymerase will be processive i.e., will stay bound to the denatured double-stranded nucleic acid template for at least 25 nucleotides, at least 50 nucleotides, at least 100 nucleotides, usually at least 500 nucleotides, and may be processive for at least 1000 nucleotides or more.

Preferably, the polymerase used in the method disclosed herein possesses high replicative fidelity. Accordingly, this polymerase will faithfully the correct, matched nucleotides along the nucleic acid template. In particular, the polymerase will incorporate the nucleotide analogue in regard of its complementary base, but will not misincorporate nucleotides. A polymerase with high replicative fidelity may notably be a polymerase with a 3'-5' exonuclease activity. As used herein, "3'-5' exonuclease activity" refers to the capability of an enzyme to remove incorporated nucleotides from the 3' end of a DNA polymer. Examples of such enzymes include e.g., T4 DNA Polymerase, T7 DNA Polymerase, DEEP VENT DNA polymerase, E. coli polymerase III, Φ29 DNA Polymerase, E. coli DNA Polymerase I, E. coli DNA Polymerase I, Klenow Fragment, Phusion^{®} High Fidelity DNA Polymerase, Phusion^{®} Hot Start High Fidelity DNA Polymerase, Phire^{®} Hot Start DNA Polymerase, 9°Nm DNA Polymerase, Herpes Simplex Virus Type 1 DNA Polymerase, etc. However, polymerases lacking a such a proofreading activity, including, e.g., Manta polymerase (a Bst DNA large fragment polymerase), present a low rate of misincorporation, as observed by the present inventors, and are thus suitable for use in the present method.

The polymerase of the present disclosure may have a strand displacement activity. By "strand displacement", it is herein meant the ability for the polymerase to displace the downstream nucleic acid during synthesis. A polymerase having strand-displacement activity may be used in particular when the polymerisation reaction of step b) is performed on a nicked double-stranded nucleic acid molecule, more particularly without prior denaturation, even more particularly at temperatures comprised between 25°C and 37°C. Examples of polymerases having a strand displacement activity include notably Φ29 DNA Polymerase, Klenow Fragment (3'→5' exo-), Vent^{®} (exo-) DNA Polymerase, Deep Vent^{®} (exo-) DNA Polymerase, Deep Vent^{®} DNA Polymerase and Bst DNA Polymerase, Large Fragment (e.g., Manta^{®}).

Polymerases which can be used in the present methods are well known in the art (see e.g., Chen. Front Microbiol. 5: 305, 2014). They include, for example, Pyrococcus furiosus (Pfu) DNA polymerase (Lundberg et al, 1991, Gene, 108: 1), E. coli DNA polymerase I (Lecomte and Doubleday, 1983, Nucleic Acids Res. 11 :7505), T7 DNA polymerase (Nordstrom et al, 1981, J. Biol. Chem. 256:3112), Thermus thermophilus (Tth) DNA polymerase (Myers and Gelfand 1991, Biochemistry 30:7661), Bacillus stearothermophilus DNA polymerase (Stenesh and McGowan, 1977, Biochim Biophys Acta 475:32), Thermococcus litoralis (Tli) DNA polymerase (also referred to as Vent DNA polymerase, Cariello et al, 1991, Nucleic Acids Res, 19: 4193), Thermotoga maritima (Tma) DNA polymerase (Diaz and Sabino, 1998 Braz J. Med. Res, 31 : 1239), Thermus aquaticus (Taq) DNA polymerase (Chien et al, 1976, J. Bacteoriol, 127: 1550), Pyrococcus kodakaraensis KOD DNA polymerase (Takagi et al, 1997, Appl. Environ. Microbiol. 63:4504), JDF-3 DNA polymerase (Patent application WO 0132887), and Pyrococcus GB-D (PGB-D) DNA polymerase (Juncosa-Ginesta et al, 1994, Biotechniques, 16:820). Other examples of polymerases which can be used in the present method include E. coli DNA Polymerase III, a Bst DNA Polymerase Large Fragment (e.g., Manta^{®}), Bsu DNA Polymerase, Crimson Taq DNA Polymerase Large Fragment, Deep Vent^{®}, DNA Polymerase, Deep Vent^{®} (exo-) DNA Polymerase, E. coli DNA Polymerase I Klenow Fragment (3'→5' exo-), DNA Polymerase I Large (Klenow) Fragment, LongAmp^{®} Taq DNA Polymerase or Hot Start, M-MuLV Reverse Transcriptase, OneTaq^{®} DNA Polymerase or Hot Start, Φ29 DNA Polymerase, Phusion^{®} Hot Start Flex DNA Polymerase, Phusion^{®} High-Fidelity DNA Polymerase, Q5^{®} + Q5^{®} Hot Start DNA Polymerase, Sulfolobus DNA Polymerase IV, T4 DNA Polymerase, Taq (exo-) DNA Polymerase, Therminator^{™} DNA Polymerase, VentR^{®} DNA Polymerase, VentR^{®} (exo-) DNA Polymerase, 9° Nm DNA Polymerase, Herpes Simplex Virus Type 1 DNA Polymerase, and any combination thereof.

Preferably, the DNA polymerase is selected in the group consisting of: Φ29 DNA polymerase, *Bst* DNA Polymerase, DeepVent, OmniAmp polymerase, SD polymerase, *Bst* DNA Polymerase Large Fragment (e.g., Manta^{®}), *Bst* 2.0 DNA Polymerase, *Bst* 3.0 DNA Polymerase, and *Bsu* DNA Polymerase Large Fragment.

The polymerase activity of any of the above enzymes can be determined by means well known in the art.

Replication of the nucleic acid molecule of interest can be performed according to any method known in the art. As will be clear to the skilled person, the conditions of the replication reaction will be chosen in accordance with the properties of the polymerase selected. Conversely, the person skilled in the art will know how to select the polymerase best suited to the conditions of the replication reaction.

For example, a polymerase having a strand-displacement activity will be useful when replication is initiated from a nick in a double-stranded nucleic acid (e.g. a nicked dsDNA molecule). A nicked double-stranded nucleic acid can be generated for example by ligating at least one nicked hairpin to at least one of the ends of the nucleic acid molecule of interest, as is described further below.

In another instance, a double-stranded nucleic acid molecule (e.g., a dsDNA molecule) can be denatured at high temperatures (e.g., a temperature comprised between 80°C and 110°C, preferably between 90°C and 100°C, more preferably at 95°C) before being replicated at a temperature high enough to prevent renaturation of the original molecule without preventing hybridisation of a primer. The skilled person will advantageously use a thermostable polymerase for this type of reaction. In this case, the primer may be provided by the 3'-OH extremity of a hairpin whose stem remains hybridised at the reaction temperature.

In addition, a reverse transcriptase will be used for replicating an RNA template, as will be clear to the skilled person.

Replication of the nucleic acid molecule of interest as disclosed herein will yield a nucleic acid molecule which is double stranded and which comprises at least one modified nucleotide in one the two strands, i.e., the strand which has been synthesised. It is understood that the nucleic acid molecule obtained or obtainable by this replication step is also an object of the present disclosure. Moreover, the present disclosure also relates to the population of double-stranded nucleic acid nucleic acid molecules containing at least one modified nucleotide in one of the two strands which are obtained or obtainable by this replication step. In other words, the present disclosure provides a library of double-stranded nucleic acid molecules containing at least one modified nucleotide in one of the two strands.

Replication generates a double-stranded nucleic acid molecule, whose newly synthesised strand comprises the modified nucleotide. Since this nucleotide analogue is not naturally present in the native nucleic acid molecule of interest, detection of this nucleotide analogue does not interfere with the detection of native epigenetic modifications. It is thus possible to identify the nucleic acid molecule of interest and, at the same time, to identify the epigenetic modifications carried by the molecule, thereby increasing the density of information which can be obtained from a single molecule.

Thus in a preferred aspect, the method disclosed herein comprises a further step of detecting at least one epigenetic modification.

In a preferred embodiment, the modified base is selected in the group constituted by 5-methylcytosine (5mC), 5-hydroxymethylcytosine (5hmC), 5-formylcytosine (5fC) 5-carboxylcytosine (5caC), 5-hydroxymethyluracil (5hmU), and N6-methyladenosine (m6A). In a more preferred embodiment, the base is chosen between 5mC and 5hmC. In an even more preferred embodiment, the base is 5mC.

### Hairpin preparation

In the method disclosed herein, nucleotide analogues are inserted by the polymerase during replication of the nucleic acid molecule of interest. Detection of these nucleotide analogues in the replicated strand can then be achieved by any of the methods known to the skilled person (for a review, see e.g., Rahman et al. Micromachines (Basel). 13(6): 968, 2022). Preferably, the nucleotide analogues are detected in the replicated strand using the technology developed by the present inventors, i.e., the so-called MAGNA^{™} technology (Ding et al. Nat. Methods 9: 367-372, 2012; Wang et al. Commun Biol 4: 128, 2021; WO 2011/147931; WO 2011/147929; WO 2013/093005; WO 2014/114687).

Although the nucleic acid molecules which have been duplicated and whose newly-synthesised strand contain at least one nucleotide analogue can be used as such in any of the detection methods known to the skilled person, it may be advantageous to engineer these molecules in order to facilitate the detection of the nucleotide analogue.

Preferably, engineering these molecules comprise adding at least one hairpin to at least one end of the nucleic acid molecule of interest, prior to step a).

A "hairpin" is a double helix wherein the 5' end of one strand is physically linked to the 3' end of the other strand through an unpaired loop. The physical link can be either covalent or non-covalent. Preferentially, the physical link is a covalent bond. Thus, a hairpin consists of a double-stranded stem and an unpaired single-stranded loop.

In order to facilitate the addition of the hairpin, it may be advantageous to add one or more As at the 3' end(s) of the nucleic acid molecule before ligating the hairpin to the molecule. Commercial kits for A-tailing are available and may be used for this purpose in accordance with the manufacturer's instructions.

It must be stressed that the ligation of a hairpin to the nucleic acid molecule of interest will yield another, longer hairpin. For example, when the nucleic acid molecule of interest is double stranded, ligation of the hairpin leads to the formation of a phosphodiester bond between the 5' end of the hairpin and the 3' end of a first strand of the molecule and of another phosphodiester bond between the 3' end of the hairpin and the 5' end of the second, complementary strand. In this configuration, the resulting molecule comprises a loop and a long stem, which corresponds to the original hairpin stem followed by the paired region of the nucleic acid of interest. On the other hand, when the nucleic acid molecule of interest in single-stranded, only one strand of the hairpin is linked to the molecule through a phosphodiester bond. The second strand, however, hybridises to the first one, thus creating a hairpin with a long single-stranded extension.

The presence of at least one hairpin in the nucleic acid molecule to be analysed will facilitate the analysis of this molecule by the MAGNA^{™} technology. Indeed, as shown by the inventors, the hairpin thus linked to the nucleic acid molecule of interest behaves exactly like the hairpins constructed by the methods of the prior art. In particular, the hairpin of the invention is amenable to the same kind of single-molecule analysis as the hairpins of the prior art and under the same conditions (see e.g., Ding et al. Nat. Methods 9: 367-372, 2012; Wang et al. Commun Biol 4: 128, 2021; WO 2011/147931; WO 2011/147929; WO 2013/093005; WO 2014/114687).

Accordingly, the method disclosed herein may comprise a further step of ligating a first hairpin to at least a first end of the molecule prior to step a). This may require prior linearisation of the molecule if the molecule is circular. No such linearisation is required if the nucleic acid molecule of interest is already linear. It must be pointed out that molecules of particular interest in the context of this disclosure, such as, e.g., cfDNA, are known to be essentially linear.

The ligation of a hairpin at an end of the molecule prior to step a) may present the additional advantage that the hairpin comprises a free 3'-OH end. A 'free 3-OH' in that context means that the 3'-OH end of one nucleotide is not covalently linked to the 5-phosphate end of the next nucleotide by a 3'-5'-phosphodiester bond. A free 3'-OH extremity in the hairpin can be used as a primer for initiating the synthesis of a new strand by the polymerase. This can be achieved in various ways. This is the case for example when the nucleic acid of interest is single-stranded. The self-hybridisation of the ligated hairpin stem generates a primer which can then be used by the polymerase to synthesise a new strand. When the nucleic acid molecule of interest is double-stranded, the use of a hairpin comprising a nick will generate a primer after ligation of the hairpin to the molecule of interest. A hairpin comprising a nick may be generated by using, for example, two oligonucleotides, wherein the first oligonucleotide forms a hairpin with a single-stranded extension and the second oligonucleotide is complementary to this single-stranded extension.

The hairpin comprises an unpaired single-stranded loop. Advantageously, the loop does not comprise any nucleotide complementary to the modified nucleotide used in the nucleotide triphosphate mix.

The loop preferably comprises 10 nucleotides or less, preferably 9 nucleotides or less, preferably 8 nucleotides or less, preferably 7 nucleotides or less, preferably 6 nucleotides or less, preferably 5 nucleotides or less. More preferably, the loop consists of 3, 4, or 5 nucleotides. Even more preferably, the loop consists of 4 bases. In addition to the loop, the hairpin comprises a double-stranded stem. The stem comprises 100 bp or less, preferably 90 bp or less, preferably 80 bp or less, preferably 70 bp or less, preferably 60 bp or less, preferably 50 bp or less, preferably 40 bp or less, preferably 30 bp or less, preferably 20 bp or less, preferably 15 bp or less, preferably 10 bp or less. For example, the double-stranded stem more preferably consists of 8, 15, 20, 40, or 60 bp.

Preferably, the method of the disclosure comprises ligating more than one hairpin to the nucleic acid molecule of interest prior to step a). In other words, it may be preferable to ligate a hairpin at each end of the nucleic acid molecule. Ligating two hairpins to the nucleic acid molecule of interest can notably be used to ensure that each of the strand will be replicated in the presence of the modified nucleotide. This will result in a signature which includes information relating to the position of the nucleotide analogue on each strand, i.e., the density of information is twice as high as with a molecule ligated to a single hairpin.

Accordingly, the present method may further comprise a step of ligating a first hairpin to a first end of the nucleic acid molecule and another step of ligating a second hairpin at a second end of the nucleic acid molecule prior to step a).

The second hairpin may or may not comprise a nick. Using a second hairpin with a nick allows quick analysis of the replicated strand. It is thus an easy and quick way for identifying the nucleic acid molecule of interest. On the other hand, when the second hairpin does not comprise a nick, both replicated strands are present on the same molecule. In a single reaction it is thus possible to detect the positions of the nucleotide analogue on both strands. Both strands will be analysed together, ensuring that all bases and their complementary bases will be analysed. Meanwhile, epigenetic modifications will still be analysable on the native strand.

The first and second hairpin may be identical or different. Ligating a second hairpin which is identical to the first hairpin leads to a homogeneous population of ligated molecules. The information obtained from the starting material is maximised since there is no loss. Alternatively, the second hairpin may be different from the first hairpin. This is useful for identifying the resulting strands during analysis. For example, if there is no nick present in the second hairpin, replication will yield the original two strands of the molecule of interest, both comprising the incorporated modified nucleotide, separated by the second hairpin. If the sequence of the second hairpin is known, then it is possible to identify the beginning and the end of each strand of the molecule of interest. Preferably, the loop of the second hairpin does not comprise a nucleotide complementary to the modified nucleotide triphosphate used in the nucleotide triphosphate mix.

When the second hairpin is different, it is advantageous to use a hairpin having a bigger loop than the first hairpin. The loop preferably comprises 50 nucleotides or less, preferably 45 nucleotides or less, preferably 40 nucleotides or less, preferably 35 nucleotides or less, preferably 30 nucleotides or less, preferably 25 nucleotides or less. More preferably, the loop consists of 10, 15, or 20 nucleotides. Even more preferably, the loop consists of 15 bases. In addition to the loop, the hairpin comprises a double-stranded stem. The stem comprises 100 bp or less, preferably 90 bp or less, preferably 80 bp or less, preferably 70 bp or less, preferably 60 bp or less, preferably 50 bp or less, preferably 40 bp or less, preferably 30 bp or less, preferably 20 bp or less, preferably 15 bp or less, preferably 10 bp or less. For example, the double-stranded stem more preferably consists of 8, 15, 20, 40, or 60 bp.

### Detection of modified nucleotides

Detection of these nucleotide analogues in the replicated strand can be achieved by any of the methods of single-molecule analysis known to the skilled person, including optical methods, electrical methods, force-based methods, combinatorial integrated methods, etc. In particular, the skilled person may use microfluidic and optofluidic platforms, including, but not limited to, bio-imaging, adaptive optical lenses, optofluidic microscopy, lasers, biological and chemical sensing, energy harvesting, and particle manipulation; near-field scanning optical microscopy (NSOM), photoactivated localisation microscopy (PALM), stimulated emission depletion (STED), stochastic optical reconstruction microscopy (STORM), fluorescence-based single-molecule methods, on-chip sensing platforms, single-particle sorting methods, single-molecule trapping methods, single-molecule analysis electrical methods, nanopores, nanowire platforms, molecular junctions, carbon-nanotube-based platforms, field-effect transistor based platforms, atomic force microscopy (AFM), optical tweezers, magnetic tweezers, etc. (for a review, see e.g., Rahman et al. Micromachines (Basel). 13(6): 968, 2022).

Preferably, the nucleotide analogues are detected in the replicated strand using a technology using magnetic tweezers, more preferably the technology developed by the present inventors, i.e., the so-called MAGNA^{™} technology (Ding et al. Nat. Methods 9: 367-372, 2012; Wang et al. Commun Biol 4: 128, 2021; WO 2011/147931; WO 2011/147929; WO 2013/093005; WO 2014/114687).

Accordingly, the detection of the position of the modified nucleotide in the nucleic acid molecules in the step c) of the present method relies on the use of a protein binding specifically to the modified nucleotide. In particular, the detection of the binding of this protein to the modified nucleotide indicates precisely the physical localisation of the nucleotide analogue in the nucleic acid molecule resulting from the replication reaction of step b).

Accordingly, the method disclosed herein comprises the steps of:
a) contacting a nucleic acid molecule with a polymerase and a mix of nucleotides triphosphates, wherein at least one of the nucleotides triphosphates is a modified nucleotide triphosphate,
b) performing a replication reaction with the polymerase and the nucleotides triphosphates mix of step a), and with the nucleic acid molecule as template,
c) detecting the position of the modified nucleotide in the nucleic acid molecules obtained by the reaction of step b), thereby generating a unique signature,
   wherein detecting the position of the modified nucleotide comprises the step of detecting the binding of a protein to the modified nucleotide in the molecules obtained by the reaction of step b);
   and
d) identifying the nucleic acid molecule based on the unique signature of step c).

By 'detecting the binding of a protein to a nucleic acid molecule', it is herein meant all the activities leading directly or indirectly to the obtainment of some information on the presence or absence of an interaction between the protein and the nucleic acid molecule. The detection of the binding may or may not involve the determination of additional information, such as e.g., the kinetic parameters of the binding reaction or the sequence of the site bound by the protein. As will be apparent to the person of skill in the art, the present method allows for such determination to be performed easily.

The technology developed by the inventors is based on the observation that the two strands of a denatured double-stranded nucleic acid will re-hybridise under appropriate conditions (see e.g., Ding et al. Nat. Methods 9: 367-372, 2012; Wang et al. Commun Biol 4: 128, 2021; WO 2011/147931; WO 2011/147929; WO 2013/093005; WO 2014/114687). If a molecule is bound to any of the strands of the denatured double-stranded nucleic acid molecule during the renaturation step, the rehybridisation will only be partial. Under certain conditions, this pause in rehybridisation, be it permanent or transient, can be used to detect an interaction between a protein and the denatured double-stranded nucleic acid molecule. Accordingly, it is possible to detect a blockage of the re-hybridisation of the double-stranded nucleic acid molecule; the physical parameters (e.g., the duration of the blockage, the position of the blockage on the double-stranded nucleic acid molecule) associated with this blockage then allow the detection of an interaction between a protein and the sequence of the nucleic acid.

This technology can be readily applied to the analysis of the nucleic acid molecule of interest of the present method. As explained above, these molecules are ligated to at least one hairpin, thereby yielding bigger hairpin which generate hairpins after the replication reaction of step b).

The present method thus comprises a step of determining the binding of a protein to the modified nucleotide in a nucleic acid molecule, wherein this step comprises a step of detecting a blockage of the renaturation of a denatured double stranded nucleic acid molecule.

There are several possibilities known to the skilled person to denature the nucleic acid. In a most preferred manner, the two strands are separated by submitting them to a physical force. A 'physical force' according to the disclosure is any influence that causes an object to undergo a certain change, either concerning its movement, direction, or geometrical construction. It will be clear to the skilled person that a force according to the invention is different from other physical parameters such as e.g., temperature (which is a direct property of matter rather than an influence exerted thereon). Physical forces according to the invention comprise such forces as friction, tension, normal force, air resistance force, applied force, and elastic force. Most preferably, the physical force according to the invention is a tension force. Accordingly, the free ends of the double-stranded nucleic acid may be pulled apart, thus rupturing all the bonds between the paired bases, and opening the double-stranded nucleic acid.

The MAGNA^{™} technology (Ding et al. Nat. Methods 9: 367-372, 2012; Wang et al. Commun Biol 4: 128, 2021; WO 2011/147931; WO 2011/147929; WO 2013/093005; WO 2014/114687) can be applied to any type of double-stranded nucleic acid. Most often, the double-stranded nucleic acid will be DNA, but it is understood that the invention also applies to single-stranded DNA-single-stranded DNA duplexes, perfectly paired or not perfectly paired, or alternatively to single-stranded DNA-single-stranded RNA duplexes, perfectly paired or not perfectly paired, or alternatively to single-stranded RNA-single-stranded RNA duplexes, perfectly paired or not perfectly paired. Furthermore, the duplex may consist of at least partial re-pairing of two single strands obtained from samples of different origins. Finally, the technology can also be applied to the secondary structures of a sole single-stranded DNA or of a sole single-stranded RNA.

Preferably, the MAGNA^{™} technology is used to detect the specific binding of a protein to the modified nucleotide present in the newly-synthesised strand of the molecule of step b).

Thus, the method of the disclosure comprises the detection of the binding of a protein to a modified nucleotide in a nucleic acid molecule, wherein the detection comprises the steps of:
(i) denaturing the double-stranded nucleic acid molecule by applying a physical force to the molecule; and
(ii) detecting a blockage of the renaturation of the double-stranded nucleic acid.

Advantageously, the detection comprises the further step of determining the position of the blockage.

In this type of method for assaying the binding of a protein to a DNA molecule, it can be advantageous, in order to facilitate re-pairing, to arrange for the free ends of the double-stranded DNA molecule (i.e., the ends which are not attached to supports) to be joined to one another covalently or quasi-covalently before pulling apart. Preferably, the double-stranded nucleic acid molecule is a hairpin, such as the one obtained by the replication reaction of step b) of the nucleic acid molecule of interest ligated to a hairpin, as described above. If it is desired that the double-stranded nucleic acid be represented diagrammatically in the context of the present disclosure, it is possible to liken it to a "zip fastener", which is opened (or closed): the denaturation of the double-stranded nucleic acid is the unzipping, the renaturation the re-zipping.

Under certain conditions, when a molecule (e.g., a protein binding specifically to a nucleotide analogue present in the nucleic acid) is bound to the denatured double-stranded nucleic acid molecule, renaturation of the double-stranded nucleic acid molecule is blocked. The molecule bound can be of any type of molecule with an affinity for a specific sequence on the denatured double-stranded nucleic acid molecule, e.g., a nucleic acid, a protein or a small molecule.

Preferably, a protein is used to block the renaturation of the double-stranded nucleic acid. More preferably, this protein is a protein that binds specifically the nucleotide analogue of step a) of the present method. This protein can be for example an antibody that specifically recognises and binds to the nucleotide analogue. This antibody is more preferably a monoclonal antibody. For example, monoclonal antibodies are available commercially against 5-bromo-2'-deoxyuridine-5'-triphosphate; 5-iodo-2'-deoxyuridine-5'-triphosphate (OTI2F8, from Abcam, Cambridge, UK); pseudouridine-5'-triphosphate (APU-6, from MBL, Woburn, AAA), etc.

Antibodies against modified nucleotides can be generated by any means known to the skilled person. An antibody reactive with a nucleotide analogue can notably be generated by recombinant methods such as selection of libraries of recombinant antibodies in phage or similar vectors, or by immunising an animal with the antigen or an antigen-encoding nucleic acid. Specificity of the generated antibodies can then be assessed by verifying that the antibodies bind the nucleotide analogue but do not bind the corresponding natural nucleotide (See, Borrebaeck (ed.) (1995) Antibody Engineering, Second Ed., Oxford University Press.; Kuby (1997) Immunology, Third Ed., W.H. Freeman and Company, New York).

It may be advantageous in some cases to use an antigen-binding fragment (such as, for example, Fab', F(ab')₂, Fab, Fv, rIgG, and scFv fragments) which is capable of specifically binding to the nucleotide analogue in the method disclosed herein. Monovalent antigen-binding fragments are particularly useful. In particular, the full-length antibody or a divalent antigen-binding fragment thereof will bind two contiguous or closely spaced modifications are, the antibody will bind both at the same time. It will then be difficult to determine precisely the respective positions of these two modifications. In contrast, monovalent antibody fragments such as scFvs can bind contiguous bases more efficiently than full-length antibodies thereby providing accurate information on the nucleic acid molecule of interest.

Accordingly, the detection of the binding of a protein to the modified nucleotide in the molecules obtained by the reaction of step b) comprises the steps of:
(i) denaturing the double-stranded nucleic acid molecule obtained by the reaction of step b) by applying a physical force to the molecule;
(ii) providing a protein capable of binding specifically to the modified nucleotide;
(iii) renaturing the double stranded nucleic acid molecule in the presence of the protein and
(iv) detecting a blockage of the renaturation of the double-stranded nucleic acid.

Advantageously, the detection comprises the further step of determining the position of the blockage.

In a typical configuration, the double-stranded nucleic acid molecules may be specifically anchored on two solid substrates (e.g., microscope slide, micropipette, microparticle). One of the ends may be attached directly or indirectly to a support, while the other end is attached directly or indirectly to a movable support. In this embodiment, a tension is applied on both ends of the double-stranded nucleic acid when the supports are moved away. When the tension is higher than a threshold value, the two strands are separated and the nucleic acid molecule is denatured. The tension applied is preferentially above or equal to 15 pN; it is more preferentially above or equal to 16 pN; it is even more preferentially above or equal to 17 pN; in a very much preferred aspect, it is above or equal to 18 pN. This force may vary with temperature, nucleotide type and buffer, but the skilled person will easily adapt the force with regard to these parameters in order to obtain the separation of the two strands. On the other hand, when the tension is decreased under a minimal value, the two strands of the denatured double-stranded nucleic acid can re-hybridise. To obtain rehybridisation of the two strands, a tension of less than or equal to 12 pN is preferentially applied; more preferentially, it is less than or equal to 11 pN; even more preferentially, it is less than or equal to 10 pN.

As mentioned above, the ligation of nucleic acid molecule of interest to at least one hairpin yields a bigger hairpin. Replication of this structure in step b) generates hairpins, which may then be anchored to two solid substrates, as described above.

A hairpin consists of a double-stranded stem and an unpaired single-stranded loop. In a hairpin, the ends of the two strands which are not engaged in the loop are free and can thus be pulled apart. This results in the unpairing of the double stranded nucleic acid, thus yielding a denatured double stranded nucleic acid molecule. It is possible to open completely a hairpin double-stranded nucleic acid molecule by pulling on each end of the nucleic acid molecule with a force higher than a threshold value. When the tension applied to the molecule is decreased to less than a minimal value, the nucleic acid molecule re-hybridises to reform a hairpin. The presence of a protein bound to the denatured nucleic acid molecule leads to a pause in re-hybridisation. Therefore, the detection of a change in the duration of such a pause indicates that a protein is bound to at least part of the double-stranded stem.

It is advantageous in this respect to design the loop sequence and length so that the hairpin refolds after a short transient, e.g., 1 second. Methods to this effect have been described in the prior art, e.g., in Woodside et al., Proc. Natl. Acad. Sci. U.S.A., 103 (16): 6190-6195, 2006). When the force is decreased from the opening to the test value, the extension of the open hairpin varies because of the elasticity of single stranded DNA. The small delay before the hairpin refolds allows the user to determine the hairpin extension at the same force than the one used to detect the blocking state.

Using a hairpin makes it possible, in particular, to perform cycles of pairing and unpairing and thus to improve the signal/noise ratio.

Techniques allowing the free ends of double-stranded nucleic acid to be joined together are known, and some will be described in greater details in what follows.

By determination of the blockage, it is herein meant the determination of the physical parameters associated with the blockage. One useful parameter is the position of the blockage on the double-stranded nucleic acid molecule, the position corresponding to the position of binding of the protein to the opened double-stranded nucleic acid molecule. Indeed, the inventors have found that the position on the double-stranded nucleic acid at which the pause in renaturation occurs can be precisely determined: the use of a hairpin affords the skilled person a means to determine the physical distance between the two free ends of the hairpin at any time during the denaturation/renaturation process.

Thus, it is particularly advantageous that the detection of the binding of a protein to a modified nucleotide in a nucleic acid molecule in the method disclosed herein comprises a further step of determining the position of the blockage.

According to this preferred instance, the disclosure provides a method for the determination of the binding of a protein to a modified nucleotide in a nucleic acid molecule, the method comprising the steps of:
(i) denaturing the double-stranded nucleic acid molecule by applying a physical force to the molecule;
(ii) providing a protein capable of binding the modified base;
(iii) renaturing the double stranded nucleic acid molecule in the presence of the protein;
(iv) detecting a blockage of the renaturation of the double-stranded nucleic acid; and
(v) determining the position of the blockage on the double-stranded nucleic acid molecule.

By 'free end' it is herein meant the end of one strand which is not covalently linked to an extremity of the other strand; as explained above, these free ends may each be bound to a different surface. For example, one of these surfaces may be movable, whilst the other may be motionless. The skilled person will thus easily realise that, in order to measure the distance between the free ends of the hairpin double-stranded nucleic acid, it is possible to simply measure the distance between the two surfaces.

This distance is maximal (z_{high} (Fₒₚₑₙ)) when the hairpin molecule is completely denatured, since the hairpin nucleic acid is then completely extended; it is minimal (z_{low} (Fₜₑₛₜ)) when the hairpin molecule is completely renatured. It is advantageous to perform all length comparisons at the same force Fₜₑₛₜ, so that the single stranded nucleic acid has the same elastic properties. Using the delay in loop closing the skilled user can measure z_{high} (Fₜₑₛₜ). Likewise, the distance between the two free ends when the renaturation process is temporarily paused can be measured: as expected, this distance z is comprised between z_{high} and z_{low} (all z being measured with F = Fₜₑₛₜ). It is immediately clear that the distance z varies with the localisation in the hairpin molecule of the binding site of the modified nucleotide-binding protein. If the protein is bound at a position which is close to the free ends of the hairpin, the self-rehybridisation process is blocked just before the complete hairpin is reformed; in this case, zₚₐᵤₛₑ is minimal. On the other hand, if the protein binds to a part of the hairpin which is close to the unpaired loop, the renaturation process will be arrested in a situation where the hairpin is completely, or almost completely denatured; in this case, zₚₐᵤₛₑ is maximal.

It is possible to correlate precisely a physical distance in a double-stranded nucleic acid molecule with a number of bases. For example, a distance of 0.8 nm corresponds to the distance spanned by two successive nucleotides (1 bp) in a single strand nucleic acid under a 10 pN force. The exact calibration of extension versus force is given by the elasticity of single stranded nucleic acid. Therefore, by simply measuring the distance between the two free ends of the partially re-zipped double-stranded nucleic acid molecule (or any two reference positions on the molecule), it is possible to determine precisely where the renaturation is blocked.

Thus, in one aspect, the method disclosed herein comprises a step of detecting the binding of a protein to a modified nucleotide in a double-stranded nucleic acid, wherein the double-stranded nucleic acid molecule is first denatured by application of a physical force, then re-hybridised in a presence of the protein, and the presence of a blockage in the re-hybridisation detected. In one aspect, the distance between the two ends of the partially renatured double-stranded molecule is determined when the renaturation process is blocked. Preferentially, the distance between the two ends of the molecule is determined when the molecule is completely denatured. More preferentially, the two distances are compared and the position of the blockage is determined. More preferentially, the distance between the fully extended loop and a reference hybridisation position is measured and used to determine the position of the blockage. Even more preferentially the distance between two reference hybridisation positions is measured and used to determine the position of the blockage.

The method disclosed herein is particularly useful in that the steps of binding/zipping/unzipping can be easily repeated, thereby allowing the determination of kinetics constants and improving the signal/noise ratio. The method can be repeated many times on the same molecule, thus improving the statistics and the reliability of the measurement. When proteins that bind to nucleic acids such as antibodies against modified bases are introduced into the system, their bound presence on the nucleic acid molecule can disrupt hairpin unzipping or rezipping, and the position of these transient blockages can be precisely mapped to the sequence of the hairpins, as explained above. As the process is non-destructive, the same hairpin molecules can be opened and closed many times in a single experiment. The accuracy of locating binding positions and determining binding kinetics (as explained below) increases with increasing numbers of open-close cycles.

Thus the present disclosure relates also to a method for the determination of the binding of a protein to a modified nucleotide in a nucleic acid molecule, the method comprising the steps of:
(i) denaturing the double-stranded nucleic acid molecule by applying a physical force to the molecule;
(ii) providing a protein capable of binding the modified base;
(iii) renaturing the double stranded nucleic acid molecule in the presence of the protein;
(iv) detecting a blockage of the renaturation of the double-stranded nucleic acid; and
(v) determining the position of the blockage on the double-stranded nucleic acid molecule;
wherein steps (i) to (vi) are repeated several times (so as to accumulate measurements and increase the signal/noise ratio).

Repeating the cycles of binding/zipping/unzipping is particularly advantageous because it makes it possible to determine both the on-rates of different binding ligands (based on the probability of observing the bound state), and their off-rates (from the average time of the transient hairpin blockage). This is facilitated by the robustness of the MAGNA^{™} technology. Indeed, it was reported that it is possible to open and close nucleic acid hairpin up to 10,000 times with the MAGNA^{™} technology (Wang et al. Commun Biol 4: 128, 2021).

A first parameter that can be determined is the rate of binding of the protein to the nucleic acid molecule of interest. The rate of binding as used herein refers to the number of binding events per unit of number of cycles. Advantageously, it is determined as the ratio of the number of cycles in which the binding of the protein to the nucleic acid molecule of interest is observed to the total number of cycles. It will easily be understood by the skilled person that the greater the number of iterations of steps (i) to (vi), the more accurate the determination of rate of binding of the protein to the nucleic acid of interest. Preferably, steps (i) to (vi) are repeated at least 2, at least 3, at least 4, at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 2000, at least 3000, at least 4000, at least 5000, at least 6000, at least 7000, at least 8000, at least 9000, or at least 10,000 times.

Aside from its position along the molecule, another useful parameter associated with the blockage in renaturation is the period of time during which the renaturation is blocked (referred herein as the duration of the pause in renaturation). Indeed, it is possible to measure the period of time during which the rehybridisation is blocked. For example, the skilled person can determine the period of time during which the distance between the two ends of the double-stranded nucleic acid is z as defined above, i.e. an intermediate value comprised between z_{high} and z_{low}.

The duration of the pause may also vary with the conditions of the reaction. The duration will be modulated in accordance with variations of the temperature. Likewise, the buffer conditions can also modulate the duration of the pause: for example, magnesium, betain and tetramethylammonium chloride (TMAC used at molar concentration) modulate the blocking time. However, when the temperature and the buffer are fixed, the duration of the pause will rather depend on the affinity of the protein for the nucleotide analogue.

The presence of a protein capable of binding the denatured double-stranded nucleic acid will block transiently the renaturation of the nucleic acid molecule. The duration of this blockage will also be dependent upon the affinity of the protein for the nucleic acid. It is clear that a protein with a high affinity for the molecule will lead to a longer pause than a protein with a weaker affinity.

The skilled person will immediately realise that the measurement of the pause enables the determination of the mean time of blockage and hence the kinetics parameters of the binding reaction.

Thus, in one particular aspect, the detection of the protein to the modified nucleotide in a double-stranded nucleic acid molecule comprises the steps of:
(i) denaturing the double-stranded nucleic acid molecule by applying a physical force to the molecule;
(ii) providing a protein capable of binding the modified base,
(iii) renaturing the double-stranded nucleic acid molecule in the presence of the protein; and
(iv) detecting a blockage of the renaturation of the double-stranded nucleic acid molecule, and
(v) determining the duration of the pause.

Preferably, the method comprises the further step of determining the position of the blockage.

### Detection of epigenetic modifications

Another particular application of the method disclosed herein is in the detection of epigenetic modifications. Such tests are currently very difficult to conduct and miss many DNA modifications. Yet epigenetic modifications are extremely important in a variety of pathologies including microbial infection and oncology. Advantageously, the aforementioned method can be used to screen for modifications on genomic DNA either whole or in selected regions.

Epigenetic modifications to DNA are present in the genomes of almost every living organism. Their type and location vary across organisms, tissues, and cell-types; over time; and through interaction with the environment. Some on these modifications come about through carefully controlled cellular processes. Others are the result of DNA damage.

Such modifications greatly expand the quantity of information that can be stored within DNA. For example, the *dam* gene of *Escherichia coli* encodes a DNA methyltransferase that methylates adenine in -GATC- sequences in double-stranded DNA thus regulating gene expression (see e.g., Calmann and Marinus, J. Bacterial., 185(16): 5012-5014, 2003). On the other hand, the most common epigenetic marker in eukaryotes is 5-methylcytosine (5mC). This specific modification is required to control and regulate a wide variety of important cellular and broader physiological processes and problems with DNA methylation in humans have been implicated in a variety of diseases, most notably certain types of cancer. In addition to 5mC, a wide variety of other DNA modifications exist in eukaryotes (Korlach and Turner, Curr.Opin.Struct.Biol., 22: 251-261, 2012).

As of today, the gold-standard for 5mC determination is `bisulfite conversion' where all cytosine residues are converted into uracil, except those which have been methylated or hydroxymethylated which remain unchanged (in oxidative bisulfite conversion, only the methylated cytosine residues remain unaltered). Subsequent amplification of the DNA product converts uracil into thymine. These conversion changes can then be detected through sequencing of the DNA (Song et al., Nature Biotechnol, 30(11): 1107-1116, 2012). However, this is a complicated, time consuming, and expensive process with error rates of 5-34% (Beck, Nature Biotechnol, 10: 1026-1028, 2010).

The present disclosure provides an easy method for detecting epigenetic modifications of nucleic acids without prior modification such as, e.g., bisulphite transformation. By 'epigenetic modifications', it is herein referred to modifications of the bases constituting a nucleic acid molecule which take place after the synthesis of the nucleic acid molecule. Such epigenetic modifications include, *inter alia*, 4-methylcytosine (m4C), 5-methylcytosine (5mC), 5-hydroxymethylcytosine (5hmC), 5-formylcytosine (5fC) and 5-carboxylcytosine (5caC), as well as 6-methyladenosine (m6A) in DNA and RNA, and 5-hydroxymethyluracil (5hmU) and N⁶-methyladenosine (m6A) in RNA.

Thus, in one particular aspect, the present disclosure provides a method for detecting at least one modified base comprised within a double-stranded nucleic acid molecule, the method comprising the steps of:
A. providing the double-stranded nucleic acid;
B. providing a protein capable of binding the modified base; and
C. testing the binding of the protein to the nucleic acid molecule by the method described above.

This method is particularly advantageous, because it uses unmodified binding molecules in a reversible process. For instance, when used to detect 5mC, no chemical (sodium bisulphate) reaction on the DNA is required. Moreover, the method of the invention is much more sensitive than any of the methods of the prior art, since it allows for detection of a modified base on a single-molecule basis and with great sensitivity (>95%, see e.g., Wang et al. Commun Biol 4: 128, 2021).

In a preferred instance, the modified base is selected in the group constituted by 5-methylcytosine (5mC), 5-hydroxymethylcytosine (5hmC), 5-formylcytosine (5fC) 5-carboxylcytosine (5caC), 5-hydroxymethyluracil (5hmU), and N6-methyladenosine (m6A). In a more preferred instance, the base is chosen between 5mC and 5hmC. In an even more preferred instance, the base is 5mC.

Proteins recognising and binding specifically to these modified bases have been described. For example, antibodies directed against 5mC have been described and used by staining this modification for cell-based visualisation (Ito et al., Nature, 466: 1129-1133, 2010; Ko et al., Nature, 468: 839-843, 2010; Szulwach etal., Nature Neurosci, 14: 1607-1611, 2011; Haffner et al., Oncotarget, 2: 627-637, 2011; Inoue et al., Science, 334: 194, 2011; Inoue et al., Cell Res, 21: 1670-1676, 2011). Such antibodies are commercially available (e.g., clone 33D3; ref: 39649 of Active Motif). Antibodies against 5-hydroxymethylcytosine, 5-formylcytosine, 5-carboxylcytosine, and N6-methyladenosine at least are available commercially (e.g., clone GT513; ref: MA5-42319 of Invitrogen; clone EDL FC-5; ref: MABE1092 of Sigma-Aldrich; clone D7S8U; ref: 36836 of Cell Signaling Technologies; and clone RM362; ref: MA5-33030 of Invitrogen; respectively). In this regard the skilled person can follow the teaching of Wang et al. Commun Biol 4: 128, 2021, wherein commercial antibodies were used to detect epigenetic modifications. Besides antibodies, enzymes that specifically recognise and react with the nucleotide of interest have been identified (Song et al., Nature Biotechnol, 30(11): 1107-1116, 2012). For example, the T4 bacteriophage enzyme B-glucosyltransferase (BGT) transfers a glucose moiety onto 5hmC. The Tet1-3 proteins are responsible for the conversion of 5mC into 5hmC. Methyl-CpG-binding protein 2, (MeCP2), was first identified by its affinity for DNA containing 5mC. Preferably, the protein is an antibody directed against the modified base or an enzyme specifically recognising the base. More preferably, the protein is an antibody.

The method of detecting the binding of a protein to a modified nucleotide described herein can be easily combined with a method of detection of epigenetic modifications such as the one described in e.g., WO 2014/114687. Indeed, this method for detecting epigenetic modifications uses the same apparatus as the method disclosed above. By pulling on magnetic beads tethered by a hairpin to the surface, the molecule can be unzipped. In this open state it can bound by a protein binding specifically an epigenetic modification (e.g., an antibody), which transiently block the hairpin rezipping when the pulling force is reduced. By measuring the distance from the surface to the bead of a blocked hairpin, one can determine the position of the hybrid along the molecule with nearly single-base precision, hence establishing the position of the modification in the nucleic acid. It is thus possible to identify directly the position of the modified base bound by the protein, without altering the setup of the experiment, by just replacing the buffer containing the protein binding the modified nucleotide, by a buffer suitable for binding of a protein binding to the epigenetic modification, as described in WO 2014/114687.

### Devices for assaying protein/nucleic acid interactions

Implementation of the method disclosed herein has been made possible, in particular, by the existence of devices designed for probing real-time nucleic acid interaction at the single-molecule level. Such a device is described for example in U.S. Patents Nos. 7,052,650 and 7,244,391. The apparatus described therein uses magnetic traps to apply a picoNewton scale force on a micron-sized superparamagnetic bead. Briefly, the apparatus comprises an optical microscope, magnets and a computer. The double-stranded nucleic acid molecules are anchored at multiple points at one end to a motionless element, e.g., a surface, and at the other end to a movable surface, in this case a magnetic bead. Magnets are provided for acting on the bead. In particular, the magnets may be used for pulling the bead away from the surface. However, the implementation of the method of the invention is not restricted to the above apparatus. Any device which allows one to fully extend and then refold a molecule of double stranded nucleic acid, whilst monitoring at the same time the extension of the molecule can be used to implement the method of the invention. For example, optical tweezers may be used; they require however prior force calibration and are not easily parallelized for high throughput measurements. Further drawbacks are the complexity of adjusting torsional control of the nucleic acid and the possible local heating of the solution by the focussed laser which may alter the hybridisation conditions.

The double stranded nucleic acid is incubated for a few minutes in a solution of adequate beads (for example streptavidin coated ones) to which it binds by one of its ends which is advantageously labelled (for example, with biotin). The beads can be transparent if optical tweezers are later used for manipulation or magnetic if one uses magnetic traps or tweezers for manipulation.

The bead-nucleic acid assembly is injected in a fluidic chamber the surface of which has been treated such as to bind the other end of the molecule. For example, an oligonucleotide containing a DBCO group at the 3' end can be covalently attached by copper-free click chemistry to an azide coated coverslip (such coverslips may be commercially available from PolyAn, GmbH) forming the base of the flow cell. In this realisation, the oligonucleotide attached to the flow cell can bind directly or indirectly to the second end of the molecule. The beads are thus anchored to the surface via a nucleic acid hairpin. The distance of the bead to the surface is then monitored by various means known to the person of the art: for example, the diffraction rings of their image on a camera can be used to deduce their distance, or the light intensity they scatter (or emit by fluorescence) when illuminated in an evanescent mode can be used to measure their distance. Alternatively, the magnetic field they generate can be measured (using a magnetic sensor such as GMR or Hall sensors) to deduce their distance to a sensor on the anchoring surface.

To pull on the nucleic acid molecule anchoring the beads to the surface various techniques have been described. One can use the light of a focused laser beam to trap a transparent bead near the focal point. By the relative translation of the beam with respect to the anchoring surface one can apply a force on the tethering molecule (a typical optical tweezers assay). The exerted force being proportional to the displacement of the bead from its equilibrium position, to exert a constant force on the tethering molecule requires a feedback loop on the trapping beam.

To exert a constant force on a bead, the use of the hydrodynamic drag generated by a flow around the bead has been described, but it usually yields a low spatial accuracy (> 100 nm). The preferred embodiment uses a magnetic trap to pull on superparamagnetic beads anchored to a surface by a nucleic acid hairpin as described above. In this configuration, small magnets placed above the sample are used to apply a constant force on the anchored bead, whose position can be determined with < 1 nm accuracy (depending on the pulling force and the dissipation due to hydrodynamic drag)

In every case one notices that the tethering hairpin can be mechanically fully unzipped by pulling on the beads with a force larger than about 16 pN. Reducing the tension on the molecule to below about 11 pN allows the hairpin to re-zip spontaneously (the unzipping transition is reversible though hysteretic). If, during the unzipped phase, some molecules in solution (such as proteins or complementary oligonucleotides of DNA, RNA, LNA or PNA) have bound to the stretched single stranded nucleic acid, these molecules will block the rezipping of the hairpin when the force is lowered to below 11 pN. The principle of the assay is thus to switch between two forces: a large one Fₒₚₑₙ to open the hairpin and a smaller one Fₜₑₛₜ used to allow rezipping and to measure the extension of the molecule at transient blockages. The blocking position is related to the sequence by a linear relation between full extension and the blocked one. For best accuracy, the full extension is preferably measured at the test force Fₜₑₛₜ. This is achieved by designing the hairpin loop such that it requires a fraction of a second to refold once the force is reduced from Fₒₚₑₙ to Fₜₑₛₜ .

In order to attach nucleic acids to surfaces or supports, use may be made of any one of the techniques known in the field. Essentially, the nucleic acid becomes anchored directly to the support, for example the micro-bead, which involves a functionalisation of this surface, for example by coating it with streptavidin, a COOH group, and the like, capable of reacting with the functionalised end of the nucleic acid.

Such methods necessitate, in general, functionalising the nucleic acid, especially the 3' and 5' ends, that is to say grafting appropriate chemical groups onto them. It is, moreover, preferable to join the other two free ends of the molecule by a loop in order to prevent the strands from dissociating at the end of the operation, so that the latter can be repeated if appropriate. For this purpose, different procedures may be adopted.

The simplest is to functionalise, using synthetic oligonucleotides, one of the ends of a double-stranded nucleic acid with two different functions (biotin and amine, for example), which permit anchoring to two different pre-treated surfaces. The two strands at the other end may be joined using a partially paired synthetic nucleotide in the form of a loop as described above. In this way, a paired, single-stranded nucleic acid, i.e. a hairpin, is produced from a double-stranded nucleic acid. The advantage of this method lies in its capacity to functionalise a heterogeneous population of small nucleic acid fragments (such as, e.g., cell-free nucleic acids), which can then be analysed simultaneously. This enables the two ends to be treated differently. The drawback of this method lies in the steric interference between the two adjacent functional groups, which can make coupling to the surfaces difficult. To solve this problem, it can be advantageous to add at each free end of the hairpin molecule a "spacer" sequence of bases, to the end of which a functional group is then added; the two spacer sequences are non-complementary, affording each functional group enough space to bind to its dedicated surface. More advantageously, the sequence of each spacer sequence is designed in order to use single-stranded sequencing primers of known sequence in the sequencing method of the invention. Even more advantageously, a double-stranded spacer molecule is added, preferably by ligation with a ligase, to the free ends of the double-stranded nucleic acid molecule of interest, wherein the double-stranded spacer molecule is designed such that it comprises two complementary, self-annealing sequences bordered by two single-stranded sequences. This specific design allows for differential functionalisation of each end, as illustrated in e.g., Supplementary Fig. 2 of Wang et al. Commun Biol 4: 128, 2021. The addition of a loop and/or spacers to the double-stranded nucleic acid molecules can be performed with any of the methods commonly used in molecular biology. These methods are well known to the person skilled in the art and there is thus no need to detail them here. The skilled person will notably refer to Wang et al. Commun Biol 4: 128, 2021, for examples of such methods.

As regards the actual anchoring techniques, there are many of these and they derive from the techniques for anchoring macromolecules (proteins, DNA, and the like) to commercially available pretreated surfaces. Most of these techniques have been developed for immunology tests, and link proteins (immunoglobulins) to surfaces carrying groups (--COOH, --NH₂, --OH, --N₃ and the like) capable of reacting with the carboxyl (--COOH) or amine (--NH₂) ends of proteins.

The covalent anchoring of nucleic acid may be accomplished directly, via the free phosphate of the 5' end of the molecule, which reacts with a secondary amine (Covalink --NH surface marketed by Polylabo at Strasbourg) to form a covalent bond. It is also possible to functionalise DNA with an amine group and then to proceed as with a protein. Alternatively, it is possible to attach covalently a nucleic acid containing a DBCO group at the 3' end by copper-free click chemistry to an azide coated surface (e.g., azide-coated coverslips from PolyAn, GmbH).

There are also surfaces coated with streptavidin (Dynal beads, and the like), which permit quasi-covalent anchoring between the streptavidin and a biotinylated DNA molecule.

Lastly, by grafting an antibody directed against digoxigenin onto a surface (by the methods mentioned above), a nucleic acid functionalised with digoxigenin may be anchored thereto. This represents merely a sample of the many possible anchoring techniques.

Among the attachment and anchoring techniques, there should also be mentioned, for example, the techniques described in Patent EP 152 886 using an enzymatic coupling for the attachment of DNA to a solid support such as cellulose.

Patent EP 146 815 also describes various methods of attachment of DNA to a support.

Similarly, patent application WO 92/16659 proposes a method using a polymer to attach DNA.

Naturally, the nucleic acid may be attached directly to the support but, where necessary, especially with a view to limiting the influence of the surfaces, the nucleic acid may be attached at the end of an inert arm of peptide or other nature, as is, for example, described in Patent EP 329 198.

More preferably, the nucleic acid molecule of interest comprises a single-stranded region at one end whose sequence is complementary of the sequence of an oligonucleotide attached to a support. Even more preferably, the oligonucleotide comprises a DBCO group and is attached by copper-free click chemistry to an azide-coated support (e.g., azide-coated coverslips from PolyAn, GmbH). This method of indirect attachment is notably described in Wang et al. Commun Biol 4: 128, 2021. The DBCO group can be inserted at any place within the oligonucleotide, preferably at the 3' end.

### Methods of diagnosis

The present methods can be used every time there is a need to identify small nucleic acid molecules. Because of the single-molecule resolution obtainable with the method of the invention, each molecule carrying a specific sequence can be detected and identified. Thus, the present invention affords the skilled person to numerate the number of nucleic acid molecules carrying the specific signature. The present method allows for the easy and accurate quantification of a specific nucleic acid species in a whole population of nucleic acid molecules.

In another aspect, the present disclosure thus provides a method for quantifying at least one nucleic acid molecule in a population of nucleic acids. This method comprises the steps of:
a) identifying the nucleic acid molecule using the method described above; and
b) numerating the nucleic acid molecule identified in step a).

The nucleic acid species to be quantified is a population of nucleic acid molecules, which comprise the specific signature generated in step c) of the method disclosed herein. These molecules thus differ from other nucleic acid molecules in that they contain this specific signature.

The proposed methodology does not require the use of fluorescently labelled probes and provides for additional layer of information, including the presence and position of epigenetic modifications. Applications include the fields of in vitro diagnostics, including clinical diagnostics, research in the fields of molecular biology, high throughput drug screening, veterinary diagnostics, agricultural-genetics testing, environmental testing, food testing, industrial process monitoring, biosecurity, forensics, and insurance testing. In vitro diagnostics and clinical diagnostics are related to the analysis of nucleic acid samples drawn from the body to detect the existence of a disease or condition, its stage of development and/or severity, and the patient's response to treatment. In high throughput drug screening and development nucleic acids are used similarly to other agents, such as, antigens, antibodies, receptors, etc., to analyse the response of biological systems upon exposure to libraries of compounds in a high sample number setting to identify drug leads. Veterinary diagnostics and agricultural genetics testing involve samples from a non-human animal or a plant species similar to in vitro diagnostics and to provide means of quality control for agricultural genetic products and processes. In environmental testing, organisms and their toxins that indicate the pollution of an environmental medium, e.g., soil, water, air, etc., are analysed. Food testing includes the quantitation of organisms, e.g., bacteria, fungi, etc., as a means of quality control. In industrial process monitoring, nucleic acids are detected and/or quantified to indicate proper control of a production process and/or to generate a signal if such processes are out of control. In insurance testing organisms and/or their toxins are identified in screening tests to determine the risk category of a client or to help approve candidates. There are various other applications of the detection and/or quantitation of nucleic acids and new applications are being developed constantly.

The methods disclosed herein are particularly helpful in a number of setups for establishing a diagnosis. In particular, the present method allows the quick and reliable identification of small nucleic acid molecules and thus the determination of any potential link to a pathology which could be associated with these molecules. Moreover, the identification of any epigenetic modification carried by the nucleic acid molecule leads to an additional layer of information and may help refining a diagnosis.

Cell-free nucleic acids (CNAs) include several types of DNA and RNA molecules that are present in extracellular fluids. CNAs can be isolated from a simple blood sample, which make them particularly useful as biomarkers. Indeed, CNAs are well-established biomarkers in prenatal diagnosis for the screening of genetic disorders in the foetus. They are also increasingly used as biomarkers in the diagnosis and prognosis of cancer. Finally, CNAs are promising biomarkers for the diagnosis of cardiovascular or neurological diseases and diabetes (for a review on CNAs, see e.g., Szilágyi et al. Int J Mol Sci. 21 (18): 6827, 2020).

The size of the CNAs can be heterogeneous, but about the vast majority of these molecules comprise less than 200 bp (for cfDNA) or 200 nucleotides (for cfRNA). Detection and quantification of circulating cell-free nucleic acids is still challenging, which limits their use in medicine. Problems in determination are mostly due to their short length and low concentrations in body fluids. Analysis of such molecules requires amplification prior to sequencing, which can introduce mutations and thus potentially leads to diagnosis errors. Moreover, amplification also erases all epigenetic modifications. On the other hand, no single-molecule diagnosis method based on the identification of CNAs is available.

In a first aspect, the methods disclosed herein of identification of a small nucleic acid molecule, and optionally of detection of epigenetic modifications, can be used to predict the sex of foetus. Accordingly, it is herein provided a method of detection of a nucleic acid molecule of foetal origin in a biological sample from a pregnant female, wherein the method comprises:
A. identifying nucleic acid molecule in the sample using the method disclosed herein,
wherein the nucleic acid molecule is a paternally inherited signature which is not possessed by the pregnant female.

The biological sample is preferably selected in the group consisting of body fluids, including but not limited to blood, plasma, serum, mucus, lymphatic fluid, synovial fluid, cerebrospinal fluid, saliva, amniotic fluid, amniotic cord blood, urine, vaginal fluid and semen, preferably blood, serum and plasma.

In addition, the methods disclosed herein allow the detection of foetal chromosomal abnormalities in CNAs. By "chromosomal abnormality", it is herein referred to an atypical number of chromosomes or a structural abnormality in one or more chromosomes. An atypical number of chromosomes is called "aneuploidy": it is thus the condition of having less than or more than the normal diploid number of chromosomes. Aneuploidy occurs when an individual is missing either a chromosome from a pair (monosomy) or has more than two chromosomes of a pair. A "trisomy" is an aneuploidy in which there are three copies, instead of the normal two, of a particular chromosome. By "structural abnormality in a chromosome", it is herein meant an event affecting the copy number of a portion of a chromosome, such as e.g., a deletion, a translocation, a duplication, a ring, etc.

Foetal aneuploidy is usually the result of a chromosome segregation defect during meiosis in the parental germ line. Although, foetal aneuploidy is not as common as other birth defects, affecting 9 in 1000 births, its detection has offered considerable technical challenges.

Maternal blood contains free maternal DNA and free foetal DNA, the foetal DNA ending up in the blood as a result of cellular death, shear etc. (Herzenberg et al., Proc. Natl. Acad. Sci. USA, 76: 1453-1455, 1979; Bianchi et al., Proc. Natl. Acad. Sci. USA, 87: 3279-3283, 1990). It is known that cell-free foetal DNA represents only 3-6 % of the DNA that is present in the maternal plasma (Lo et al., Am J Hum Genet, 62: 768-775, 1998). Methods have been described for diagnosing foetal aneuploidy from maternal blood; however, these methods require a step of amplification of the genetic material or use shotgun sequencing, a method with prior PCR-based enrichment (Lo, BJOG, 116: 152-157, 2009; Fan et al., Proc. Natl. Acad. Sci. USA, 105(42): 16266-16271, 2008; Chiu etal., BMJ, 342: c7401, 2011 doi: 10.1136/bmj.c7401), and are thus susceptible of potential bias.

In another aspect, the present disclosure also provides a method of detection of foetal aneuploidy in a population of nucleic acid molecules, wherein the population comprises maternal and foetal genetic material, in a biological sample from a pregnant female, comprising the steps of:
A. quantifying a nucleic acid molecule comprising a signature associated with foetal aneuploidy using the method disclosed herein;
B. quantifying a reference nucleic acid molecule, the reference nucleic acid molecule being preferably a nucleic acid molecule comprising a signature not associated with foetal aneuploidy, using the method disclosed herein; and
C. comparing the quantifications of steps A and B;
wherein foetal aneuploidy is indicated by a number of molecules in A higher than the number of molecules in B.

The number of molecules in A and the number of molecules in B may each be normalised to the total number of molecules.

Preferably, the specified chromosome portion is a chromosome portion which is suspected of being abnormally distributed in the foetus. Advantageously, the second chromosome portion is a reference chromosome portion, i.e., a chromosome portion which is not affected by an abnormal distribution; the second chromosome portion is thus preferably disomic in foetal cells.

Preferred foetal chromosomal aneuploidies and accompanying diseases or disorders which can be determined by the method of the present invention include Turner syndrome (gonosomal monosomy), Klinefelter syndrome (XXY gonosomes), triple-X syndrome (XXX gonosomes), Down syndrome (Trisomy 21), Edwards syndrome (Trisomy 18) or Patau syndrome (Trisomy 13). Uniparenteral disomy is known for chromosome 15 as Prader-Willi-Syndrome. If such a uniparenteral disomy is to be detected, the DNA must also be analysed in a way which distinguishes whether it is of maternal or paternal inheritance. Unbalanced translocations as used herein encompass, preferably, unbalanced Robertson trisomy, rob(13q;14q). Other structural aberrations which can be preferably determined by the method of the invention include 4q-deletion (Wolf-Hirschhorn syndrome), 5q-deletion (cri du chat syndrome) or microdeletion syndromes, in particular, 17ql l .2 deletion (Smith-Magenis syndrome) or 22ql 1.2 deletion (DiGeorge syndrome).

In a preferred instance, the signatures used in steps A and B are signatures specific for foetal DNA. For example, these signatures correspond to the alleles inherited from the father. In another instance, neither signature is specific for either foetal or maternal DNA. However, since 10 % of the DNA present in the blood is of foetal origin, a trisomy should result in a ratio of step C of 1.05. About 1 million beads would have to be probed (by scanning the sample) corresponding for each chromosome to an average 2 x 10⁴ sequences of which about 2 x 10³ will be of foetal origin. The expected statistical (counting) error will be about 1% which allows for large enough S/N for diagnostics.

Circulating tumour DNA (ctDNA) is emerging as one of the most promising biomarkers for early cancer detection. These are associated with different epigenetic modifications, which show disease-related variations and so finding their role as epigenetic biomarkers in clinical settings (see e.g., Rahat et al. Front Genet. 11: 844, 2020; Cirmena et al. Cancers (Basel).; 13(14): 3460, 2021). Tumours secrete ctDNA into the bloodstream before they are visible on imaging, signs of disease are detected, or both. In cancer-suffering patients, the major proportion of ctDNA is released via apoptosis or necrosis of tumour cells or through processes of active release from neoplastic cells or extracellular vesicles. The detection and analysis of ctDNA, has shown excellent correlations with solid tumour molecular pathology, so that ctDNA is now a frequently utilised, clinically accepted alternative to tissue biopsies for tumour genotyping and guiding treatment decisions in patients with metastatic cancer. In addition, evaluating patient risk profiles and integrating ctDNA assays with orthogonal multi-omic assays (including the assessment of epigenetic modifications) have the potential to further enhance the utility of ctDNA as an early cancer detection tool. In particular, cell-free 5hmC modifications may potentially be a promising and informative biomarker used not only to identify cancer types but also to track tumor stage in some cancers (Song et al. Cell Res. 27(10): 1231-1242, 2017).

However, various challenges currently limit the applicability of ctDNA to clinical practice. Problems in detection and quantification of ctDNA are mostly due to their short length and low concentrations in body fluids. As described above, these issues are obviated by the method described herein.

In another aspect, the disclosure provides a method of diagnosing a tumour in a patient, wherein the method comprises:
A. detecting the presence of a nucleic acid molecule in a biological sample from the patient using the method disclosed herein,
wherein the nucleic acid molecule comprises a tumour-specific signature.

The biological sample is preferably selected in the group consisting of body fluids, including but not limited to blood, plasma, serum, mucus, lymphatic fluid, synovial fluid, cerebrospinal fluid, saliva, amniotic fluid, amniotic cord blood, urine, vaginal fluid and semen, preferably blood, serum and plasma.

A "tumour-specific signature" as used herein is a signature as determined by the method disclosed herein which is associated with cancer. For example, a "tumour-specific signature" may be found exclusively in tumours. In other words, such a "tumour-specific signature" is not found to substantial levels in a non-cancerous tissue. Alternatively, a "tumour-specific signature" may be a signature whose presence is increased in a tumour in comparison to a non-cancerous tissue. Since epigenetic modification in ctDNA are reliable cancer biomarkers, the identification of a "tumour-specific signature" preferably comprises the detection of epigenetic modifications as described herein.

The present disclosure thus provides a method for diagnosing a cancer in a patient. Examples of cancer which can be identified according to the methods disclosed herein include but are not limited to, carcinoma, lymphoma, blastoma, sarcoma, and leukaemia or lymphoid malignancies. More specifically, a cancer according to the present disclosure is selected from the group comprising squamous cell cancer (e.g., epithelial squamous cell cancer), lung cancer including small-cell lung cancer, non-small cell lung cancer, adenocarcinoma of the lung and squamous carcinoma of the lung, oropharyngeal cancer, nasopharyngeal cancer, laryngeal cancer, cancer of the peritoneum, oesophageal cancer, hepatocellular cancer, gastric or stomach cancer including gastrointestinal cancer and gastrointestinal stromal cancer, pancreatic cancer, glioblastoma, brain cancer, nervous system cancer, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, gallbladder cancer, vulval cancer, testicular cancer, thyroid cancer, Kaposi sarcoma, hepatic carcinoma, anal carcinoma, penile carcinoma, non-melanoma skin cancer, melanoma, skin melanoma, superficial spreading melanoma, lentigo maligna melanoma, acral lentiginous melanomas, nodular melanomas, multiple myeloma and B-cell lymphoma (including Hodgkin lymphoma; non-Hodgkin lymphoma, such as e.g., low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukaemia (CLL); acute lymphoblastic leukaemia (ALL); hairy cell leukaemia; chronic myeloblastic leukaemia (CML); Acute Myeloblastic Leukaemia (AML); and post-transplant lymphoproliferative disorder (PTLD), as well as abnormal vascular proliferation associated with phacomatoses, oedema (such as that associated with brain tumours), Meigs' syndrome, brain, as well as head and neck cancer, including lip & oral cavity cancer, and associated metastases.

In a preferred embodiment, said cancer is lung cancer, lip & oral cavity cancer, oropharyngeal cancer, nasopharyngeal cancer, laryngeal cancer, prostate cancer, oesophageal cancer, gallbladder cancer, liver cancer, hepatocellular cancer, gastric or stomach cancer including gastrointestinal cancer and gastrointestinal stromal cancer, pancreatic cancer, Hodgkin lymphoma, Non-Hodgkin lymphoma, leukaemia, multiple myeloma, Kaposi sarcoma, kidney cancer, bladder cancer, colon cancer, rectal cancer, colorectal cancer, hepatoma, hepatic carcinoma, anal carcinoma, thyroid cancer, non-melanoma skin cancer, skin melanoma, brain cancer, nervous system cancer, testicular cancer, cervical cancer, uterine cancer, endometrial cancer, ovarian cancer, or breast cancer.

In a more preferred embodiment, said cancer is oesophageal cancer, liver cancer, hepatocellular cancer, gastric or stomach cancer including gastrointestinal cancer and gastrointestinal stromal cancer, pancreatic cancer, Hodgkin lymphoma, colon cancer, rectal cancer, colorectal cancer, hepatoma, hepatic carcinoma, anal carcinoma, non-melanoma skin cancer, skin melanoma, cervical cancer, uterine cancer, endometrial cancer, ovarian cancer, or breast cancer.

As explained above, chromosomal abnormalities occur during cell division, when either whole or parts of chromosomes fail to separate properly. For example, as cancerous cells progress through oncogenesis, they accumulate chromosomal abnormalities, such as deletions, translocations, gains or loss of entire chromosomes. These chromosomal abnormalities are thought to be linked to the acquisition of the cancerous phenotype and are specific for each cancer type. The more advanced the cancer, the greater number of chromosomal abnormalities. Detecting such chromosomal abnormalities is thus usually very informative about the tumour aggressiveness and the prognosis of the patient.

In this embodiment, the invention thus provides a method for detecting an abnormal distribution of a specified chromosome portion in a biological sample from a subject. More specifically, the present method further comprises the further steps of:
B. quantifying a nucleic acid molecule comprising a tumour-specific signature using the method disclosed herein;
wherein the presence of a tumour is indicated by the number of nucleic acid molecules in A.

In order to conclude to a chromosomal abnormality, it is advantageous to compare the number of nucleic acid molecule detected in A with a reference nucleic acid molecule. The reference nucleic acid molecule is a nucleic acid molecule which is not associated with cancer, i.e., a nucleic acid molecule not released from tumour cells, and whose levels are not affected in tumour samples. Preferably, the reference nucleic acid molecule is a CAN not associated with cancer

According to this instance, the method further comprises the steps of:
C. quantifying a reference nucleic acid molecule using the method disclosed herein; and
D. comparing the quantifications of steps A and B;
wherein the presence of a tumour is indicated by a number of molecules in A higher than the number of molecules in B.

The number of molecules in A and the number of molecules in B may each be normalised to the total number of molecules.

### Kits

In another aspect, the present disclosure provides kits for practicing the methods disclosed herein.

A kit according to the disclosure comprises at least:
- a mix of nucleotides triphosphates, wherein at least one of the nucleotides triphosphates is a modified nucleotide triphosphate as described herein;
- at least one polymerase as described herein;
- optionally, at least one hairpin as described herein; and
- optionally, one protein binding to the modified nucleotide.

Preferably, the kit disclosed herein further comprises a double-stranded spacer molecule comprising two complementary, self-annealing sequences bordered by two single-stranded sequences. Each of the two single-stranded sequences of the double-stranded spacer molecule may or may not be functionalised. Accordingly, in some instances the kit disclosed herein may comprise means for functionalising the single-stranded sequences, as disclosed herein.

The kit may further contain at least one of the other molecules or reagents described herein (e.g., ligase, at least one or more reaction buffers, one or more functionalising reagents, such as DBCO one or more supports such as magnetic beads or glass slides, etc.). The different components of the kit may be present in separate containers. When some components are compatible, they can be pre-combined in one container, as desired.

In addition to the above-mentioned components, the kits according to the disclosure may further comprise instructions for using said kit components, more particularly for implementing the methods of the disclosure (e.g., instructions for synthesising the double-stranded nucleic acid molecule according to the disclosure, instructions for detecting the binding of the protein to the modified nucleotide in the newly-synthesised double-stranded nucleic acid molecule, etc.).

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., Current Protocols in Molecular Biology, Eds., John Wiley & Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989).

The examples below will enable other features and advantages of the present invention to be brought out.

### EXAMPLES

**Table 1: Nucleic acid sequences used in the Examples**

| **SEQ ID NO:** | **Sequence** | **Name** |
|---|---|---|
| 1 | cggtggtctcggaattaatggac | PS2508 |
| 2 | cggtggtctcgcgcttctctcacgcgttgtcacttcgctggacggctttggact cgggcctacggtctgctactggggctcagcggccctcagggcctcggagctt | PS2509 |
| | ggcggaggcctggctagctctgtctaggcctgctcatcggctgggatccgtgc ctatccctccgtatgctggtccattaattccgagaccaccg | |
| 3 | ggctcgtctctcctggacaggaacagaggggca | PS2972 |
| 4 | tgcccctctgttcctg | PS1289 |
| 5 | tatgtacttcgtctcgctccgtcctcgatcctgtcgccccgctgcggtgtctgg cacccttgtggacgggtcccggtgctgcccttctgttgatccggtggctatcc gctggtactctgtttcatttccctcctcggccgcctttcatgtggggtcccgct cgccgatccgtggtgcggggcttagagacctgcaactgt | Seq1 |
| 6 | tatgtacttcgtctcgctcccccggcggatttcctggtcagtggtgccggttcc ccggtgtcgtcagtgttctctcggtcccctcgcatggtcgccggccgcgagtt ccgcccggaccgtccgggaggtcgctgttccggcactcgccctccgccgacg ctcctgtcggggcccactgtggcttagagacctgcaactgt | Seq2 |
| 7 | tatgtacttcgtctcgctccgctccggggatgggggggggccctgccgttgtt gctggtgtgtattctggtgtgcgttgttgcgccgtagtcgtcgcgtcccagcct ttcggtcccgagtttcgttgatccgtctggcgtttaccgtctgtgttgccttcta ccgcttcccctcctgtttgcttagagacctgcaactgt | Seq3 |
| 8 | tatgtacttcgtctcgctcccggcaccttcgcccattctgtcctttcctgatgtt cgtcttattgttcgggacgccccttccgtggtcgaggccgtccgaggctttgtt cgcgttaccgcgttttctccattcctggctcgcttgttgtcggagggtgcggtt tttacttcgtcttggtggcttagagacctgcaactgt | Seq4 |
| 9 | tatgtacttcgtctcgctccctgtccagcttccgcgatgctcgtgtcgttgacc ctccccgtaccctgccttgatctcgcgtgtcggcacgctggcctacgcttttg gatcggtgggcctatccctcgcctcttccctcgtccttgcagtccgcgctccg ttacggggtgccagccgtttgcttagagacctgcaactgt | Seq5 |
| 10 | gccgacgcttgtagtcgcagcctgtcgttagtatcgtagccagatagaaagct tgctgatccgcttaagctagatagactgtttgtctgcacagctccgaagccag aagtaccgatgatcgcctgtataaagtcgcctgtagtcgcttgtaatgtcgtt agtaagactgacgtcgtcgaactagtaagaatagaccgaagacgtcctgtaa gcaatacgatcacctgccatgcatagatgcttattgtcgatgtcactcgtcag taagtacgccgcctgtcccgcacagcttagatgccatagttcgacgaatagc tcagttgattcacgtcacgaatacgcccagtagtctagaagcctcgacgaaa tgctgactgaaagctgaagtctacgtagacagtacttgcaaacgtagaagtc ttcgatgaagatctgccgatcgttacgttagatagcccgccatgaagatgaa gatgctgtcgacttgtatgtcgctttgcagccg | |
| 11 | gccgacgcttgtagtcgcagcctgtcgttagtatcgtagccagatagaaagct tgctgatccgcttaagctagatagactgtttgtctgcacagctccgaagccag aagtaccgatgatcgcctgtataaagtcgcctgtagtcgcttgtaatgtcgtt agtaagactgacgtcgtcgaactagtgaagagcaccagaaa | PS3000 |
| 12 | agtaccgatgatcgcctgtataaagtcgcctgtagtcgcttgtaatgtcgtta gtaagactgacgtcgtcgaactagtaagaatagaccgaagacgtcctgtaag caatacgatcacctgccatgcatagatgcttattgtcgatgtcactcgtcagt aagtacgccgcctgtcccgcacagcttgaagagcaccagaaa | PS3001 |
| 13 | atacgatcacctgccatgcatagatgcttattgtcgatgtcactcgtcagtaa gtacgccgcctgtcccgcacagcttagatgccatagttcgacgaatagctca gttgattcacgtcacgaatacgcccagtagtctagaagcctcgacgaaatgc tgactgaaagctgaagtctacgtagacagaagagcaccagaaa | PS3002 |
| 14 | tgattcacgtcacgaatacgcccagtagtctagaagcctcgacgaaatgctg actgaaagctgaagtctacgtagacagtacttgcaaacgtagaagtcttcga tgaagatctgccgatcgttacgttagatagcccgccatgaagatgaagatgc tgtcgacttgtatgtcgctttgcagccggaagagcaccagaaa | PS3003 |
| 15 | gcttgcactgaganttntctcagtgc | PS1450 |
| | Where n = internal Dig-dT | |
| 16 | ntcgctgcgtagatcgccgagaatcatagatggagtgtggtgtgccttgaaa | PS2289 |
| | Where n = DBCO- G | |
| 17 | tgattctcggcgatctacgcagcgacnatgcagtgtgtcttttggtctttctgg tgctcttc | PS2290 |
| | Where n = internal azide | |
| 18 | attcgaagagcaccagaaagaccaaaagacacaaagggtcagtgctgcaa cccacttcctaatctgtcatcttctg | PS866 |
| 19 | ggaggaagagcaccagaaagaccaaaagacacaaagggtcagtgctgcaa cccacttcctaatctgtcatcttctg | PS2551 |
| 20 | cgagctagctgnttncagctagctcggaggtcaggcgtgggttggtcctcg | PS2983 |
| | Where n = internal Dig-dT | |
| 21 | tgattctcggcgatctacgcagcgacngcaagggcgagtgctcttctcatatt ggcgctaccactccacctcccgctca*t | PS2679 |
| | Where n = internal azide | |
| 22 | tgagcgggaggtggagtggtagcgccaatatgagaagagcgcagggaggga ggagtgaacccacttcctaatctgtcatcttctg | PS2681 |
| 23 | acagttgcaggtctctaagc | PS2569 |

### Example 1: Determination of the DNA polymerase capable of inserting different modified uridine triphosphate.

In a first series of experiments, we wanted to determine which DNA polymerase was capable of using modified uridine triphosphate in a polymerisation reaction.

In order to identify the proper DNA polymerase that can utilise modified uridine triphosphate, we performed an elongation assay with different DNA polymerases and various combination of nucleotide mixes, using a 22-mer primer (PS2508, SEQ ID NO:1) annealed to a 200-mer template (PS2509, SEQ ID NO:2). As the template contains a lot of instances of adenines, there are multiple positions where the polymerase must be able to incorporate uridine to be able to produce full length fill in reaction. The MANTA^{™} DNA polymerase did not show any elongation product in the absence of dTTP or dUTP, strongly suggesting that this polymerase was completely stopped at the first instance of adenine in the template and were incapable of inserting a wrong nucleotide.

On the other hand, when dTTP or dUTP was replaced with a modified dUTP, a full-length reaction product was detected for both Manta^{™} and DeepVent DNA polymerases. Hence, the Manta^{™} DNA polymerase, although lacking proofreading activity, was capable of discriminating wrong dNTP and yet capable of incorporating the modified dUTP (Figure 1).

The conditions for the fill in reaction were as follow: dATP, dGTP, dCTP and Iodo dUTP were prepared in a mix at 500µM each and used at a final concentration of 100µM final; 1µl of Manta^{™} or DeepVent polymerase were used on 10 pmoles of the 200-mer (PS2509) with the 22-mer primer (PS2508) pre-annealed to initiate the fill-in.

The incubation was done for 30min at 65 ° C for the Manta or 72 ° C for the DeepVent, in a MiniAmp thermalCycler (Applied Biosystems) with lid at 105°C to prevent evaporation.

### Example 2: Measurement of the K_{D} of different modified uridine triphosphate by the MANTA polymerase.

We then moved to determine the K_{D} of the Manta polymerase for each of the modified dUTPs. The K_{D} is defined as the concentration of the modified dUTP required to produce 50 % of fill in reaction. Because the template used in the previous example (i.e., PS2509) contains multiple instances of insertion of the modified base tested, it was too complex to calculate the K_{D}. We then produced a second, simpler template comprising only one adenine in the template followed by 15 random bases excluding adenine (PS2972, SEQ ID NO:3). The template was designed such that the 3' end of the complementary oligonucleotide tagged with FITC (PS1289, SEQ ID NO:4) ends at the base just before the adenine in the 32-base oligonucleotide. After annealing of these two oligonucleotides, there is thus only one A position where modified dUTP could be inserted (see **Fig. 2A**).

It was expected that if dCTP, dATP and dGTP were present in the reaction mix along with one of the modified uridine triphosphates, if the modified uridine was integrated by the polymerase, 16 bases would be added, thereby yielding a 32 base-long oligonucleotide tagged with FITC. By contrast, if the polymerase did not integrate the modified nucleotide, the size of the FITC-labelled oligonucleotide would not be affected and would thus remain at 16 bases. Oligonucleotides of 16 and 32 bases can be easily separated on a PAGE gel.

Polymerisation reactions were prepared with the Manta polymerase. Each reaction was supplemented with 100 µM of dATP, dCTP and dGTP as well as the modified dUTP tested at different concentrations indicated on the figure (no dTTP was added to the reaction). The modified dUTP tested were 5-bromo-dUTP, 5-iodo-dUTP, 5-fluoro-dUTP, pseudo-dUTP, 5-formyl-dUTP, 5-hydroxymethyl-dUTP, and 5-chloro-dUTP (**Fig. 1B-H**). Both the FITC-labelled and the template oligonucleotides were mixed at equimolar concentration (5 µM) and the reaction was incubated at 65°C for 2 minutes before being stop with the addition of 20 mM of ETDA. The resulting reactions were loaded on a denaturing PAGE gel and visualized with a gel documentation. Fluorescence of the reaction products were then quantified with ImageJ (National Institutes of Health).

For each modified uridine triphosphate, the ratio of fully extended product over the total amount of FITC-labelled oligonucleotide was calculated and plotted on a graph as a function of the concentration. Either a first or second order equation was derived from these points and the K_{D} was calculated as the 50% of the reaction kinetics.

**Fig. 1I** summarises all the K_{D} calculated for the different modified nucleotides. All six modified dUTP bases tested are well incorporated by the Manta polymerase. The majority of the modified nucleotides have a K_{D} similar to dTTP, except for pseudouridine which is almost twice the K_{D} of dTTP.

### Example 3: Generation of specific signatures based on insertion of modified nucleotides and their identification.

The detection of the incorporated modified nucleotide uridine by a specific antibody to generate a unique signature was then assessed.

The strategy that was used to construct the hairpin molecules is illustrated in **Fig. 2A****.** In summary, we have generated synthetic sequences where between 10 to 12 adenines were present in a 200 bases template, therefore generating hairpins molecules with 10-12 modified dUTP. We also included a constant sequence of 20 bases complementary to a universal primer (PS2569; SEQ ID NO:23) that serves to initiate synthesis. A 20-base primer oligonucleotide (PS2569) is annealed to a template oligonucleotide (Seq 1 to 5; SEQ ID NOs:5-9) and is then incubated with the Manta polymerase in the presence of dATP, dCTP, dGTP, and a modified dUTP (iodo-dUTP). After the fill-in reaction is complete, the resulting double-stranded molecule is digested at both ends with *Bsal,* thereby generating two distinct 4-base 5'overhangs. A loop (PS1450; SEQ ID NO:15) and a Y-shaped adapter (PS2289, PS2290 and PS2551; SEQ ID NOs:16, 17, and 19, respectively) are then ligated to the fragment to generate the hairpin. The hairpin is then conjugated to the bead and to the flow cell as previously described (Wang et al. *Commun Biol* **4:** 128, 2021).

Fifty different sequences with 10-12 positions where a modified uridine can potentially be inserted were generated. In order to allow for subsequent identification by antibody binding, these sequences were designed such that they all had a different distribution of A (in terms of number and spacing between them) in the template sequence (**Fig. 2B**).

The hairpin molecules produced were attached to paramagnetic 1 µm bead and injected into a flow cell where they could be captured at the surface of the flow cell via hybridisation with oligonucleotides covalently attached at the surface. The antibody (clone OTI2F8 from Abcam) against the IdU modification was injected to detect the positions where the modified bases were located. The binding of the antibody to the base modification was detected as described in Wang et al. Commun Biol 4: 128, 2021. The experimental blockages due to the antibody were extracted and fitted to the original 50 sequences to determine the identity of the molecule.

Since only the first 5 sequences out of 50 were used for this analysis, the success rate could be determined based on how many molecules were assigned to sequences 1 to 5 over all the 50 sequences. We observed that >98% of the molecules observed experimentally could be assigned to the sequence 1 to 5 from the original mix (N = 52 molecules, **Fig. 3C**).

These results thus demonstrate that it is possible to identify univocally a nucleic acid molecule by generating a unique signature for this molecule, through incorporation of a modified nucleotide followed by the specific binding of a monoclonal antibody.

### Example 4: Generation of molecules with more than one modified nucleotide.

It was then assessed whether the signature could also be generated when using two modified nucleotides.

One of the 50 long oligonucleotides described in Example 3 was used as a template to incorporate two different modified nucleotides at the same time. Both iodo-dUTP as well as pseudo-dUTP were included in the nucleotide mix (at 5µM and 95µM respectively) during the replication step to completely replace the natural base dTTP. After synthesis of the complementary strand, the hairpin was assembled and assessed with both anti-iodouridine antibody (clone OTI2F8 from Abcam) and anti-pseudouridine antibody (Cat. No. C15200236, Diagenode) (**Fig. 3A**).

The distribution of each modified base in the hairpin was determined for each modification. An equal distribution of both modified bases on the template was observed, i.e., there was as much IdU as pseudoUridine detected in the final hairpin produced. Similar results were obtained when iodo-dUTP and bromo-dUTP were used as modified bases (data not shown)

These results confirm that it is possible to integrate multiple modified bases in the same reaction and detect them efficiently.

### Example 5: Detection of four different modified bases on the same template.

Four specific antibodies against four modified cytosines (5mC, 5hmC, 5caC and 5formylC) had previously been identified (Wang et al. Commun Biol 4: 128, 2021). These monoclonal antibodies are provided by Diagenode (anti-5mC, Cat. No. C15200003 and anti-5hmC, Cat. No. C15220001-20), Abcam (anti-5caC, Cat. No. ab185492) and Cell-Signaling (anti-5formylC, Cat. No. 74178S).

A synthetic sequence of 505 base pairs (bp) was designed to get a homogenous distribution of modified cytosine and a minimum of three nucleotides between two consecutive modified cytosines. The sequence was divided into 4 different regions (**Fig. 4A**) which were used as 4 templates for hairpin constructions, as described in Example 2. Each of the template has overlapping sequence with the previous or next sequence such that it was possible to reconstruct the entire sequence with these for fragments and were used to insert four different modified cytosine bases.

The comparative kinetics of modified base incorporation by Manta polymerase was determined as described in Example 1. Except for 5caC, the kinetic of incorporation for other modified cytosine bases was similar than for the natural dCTP (**Fig. 5B**). This experiment allowed to adjust the concentration of modified cytosines to get around 25% of incorporation of each modified base per template in the final hairpins. The mix of dNTP used contains 100 µM of each dATP, dGTP, dTTP and 5ca-dCTP, 20 µM of 5hm-dCTP and 10 µM of each 5m-dCTP and 5f-dCTP. The difference in the concentration of each modified cytosine reflects how well they can be used by the Manta polymerase according to their calculated K_{D}. This mix of dNTP enable us to obtain an equivalent distribution of modified cytosines (22.7% for 5hmC, 20.45% for 5caC, 22.7% for 5fC, and 27.2% for 5mC) detected by specific antibodies (**Fig. 5D**). The positions detected by each antibody are shown in **Fig. 5C**.

## Claims

1. A method of identifying a nucleic acid molecule in a population of nucleic acid molecules, the method comprising the steps of:
a) contacting a nucleic acid molecule with a polymerase and a mix of nucleotides triphosphates, wherein at least one of the nucleotides triphosphates is a modified nucleotide triphosphate,
b) performing a replication reaction with the polymerase and the nucleotides triphosphates mix of step a), and with the nucleic acid molecule as template,
c) detecting the position of the modified nucleotide in the double-stranded nucleic acid molecules obtained by the reaction of step b), thereby generating a unique signature, and
d) identifying the nucleic acid molecule based on the unique signature of step c).

2. The method of claim 1, wherein the modified nucleotide is selected in the group consisting of nucleotides incorporating bases such as 3-nitropyrrole 2'-deoxynucloside 5-nitroindole 2'-deoxynucleoside, alpha phosphorothiolate, phosphorothioate, pyrazolo[3,4-d]pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyl uracil and cytosine, 6-azo uracil, cytosine and thymine, 5F-uracil (pseudouracil), 4-thiouracil, 8-halo (e.g., 8-bromo), 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, deazaguanine, 3-deazaguanine, 3-pyrazolo[3,4-d]pyrimidine, imidazo[1,5-a]1,3,5 triazinones, 9-deazapurines, imidazo[4,5-d]pyrazines, thiazolo[4,5-d]pyrimidines, pyrazin-2-ones, 1,2,4-triazine, pyridazine; and 1,3,5 triazine; the modified nucleotide being preferably selected in the group consisting of 5-bromo-2'-deoxyuridine-5'-triphosphate, 5-iodo-2'-deoxyuridine-5'-triphosphate, 5-fluoro-2'-deoxyuridine-5'-triphosphate, 5-ethynyl-2'-deoxyuridine-5'-triphosphate, 5-chloro-2'-deoxyuridine-5'-triphosphate, 5-methyluridine triphosphate, 5-hydroxymethyluridine-5'-triphosphate, 1-methyl-pseudouridine-5-triphosphate, 1-methyl-pseudouridine-phosphoramidite, pseudouridine-5'-triphosphate, 5-formyl-2'-deoxyuridine-5'-triphosphate, 5-carboxy-2'-deoxyuridine-5'-triphosphate, 5-fluorouridine-5'-triphosphate; 5-methyluridine (m5U), 5-taurinomethyluridine (τm5U), 5-carboxymethylaminomethyluridine (cmnm5U), 5-hydroxyuridine (ho5U), 5-methoxyuridine (mo5U), 5-carboxymethyluridine (cmo5U), 5-carboxymethoxyuridine (mcmo5U), 5-bromo-2'-deoxycytidine-5' triphosphate, 5 iodo 2' deoxycytidine 5' triphosphate, 5 fluoro 2' deoxycytidine 5' triphosphate; the modified nucleotide being more preferably in the group consisting of 5-bromo-2'-deoxyuridine-5'-triphosphate, 5-iodo-2'-deoxyuridine-5'-triphosphate, 5-fluoro-2'-deoxyuridine-5'-triphosphate, pseudouridine-5-triphosphate, 5-formyl-2'-deoxyuridine-5'-triphosphate, 5-carboxy-2'-deoxyuridine-5'-triphosphate, 5-hydroxymethyl-2'-deoxyuridine-5'-triphosphate, and 5-chloro-2'-deoxyuridine-5'-triphosphate.

3. The method of any one of claims 1 or 2, wherein step c) comprises contacting the nucleic acid molecule with a protein capable of binding specifically the modified nucleotide triphosphate, preferably with an antibody capable of binding specifically the modified nucleotide triphosphate.

4. The method of any one of claims 1 to 3, comprising a further step of detecting epigenetic modifications, preferably 5-methylcytosine (5mC), 5-hydroxymethylcytosine (5hmC), 5-formylcytosine (5fC) 5-carboxylcytosine (5caC), 5-hydroxymethyluracil (5hmU), or N6-methyladenosine (m6A), more preferably 5mC or 5hmC, in the nucleic acid molecule.

5. The method of claim 4, wherein the further step of detecting epigenetic modifications in the nucleic acid molecule comprises contacting the nucleic acid molecule with a protein capable of binding specifically the epigenetic modification, preferably with an antibody capable of binding specifically the epigenetic modification.

6. The method of any one of claims 1 to 5, comprising a step of ligating at least one hairpin to at least one end of the molecule of interest, prior to step a).

7. The method of any one of claims 1 to 6, wherein the detection of step c) comprises the steps of:
(i) denaturing the double-stranded nucleic acid molecule by applying a physical force to the molecule;
(ii) providing a protein capable of binding the modified nucleotide triphosphate;
(iii) renaturing the double stranded nucleic acid molecule in the presence of the protein;
(iv) detecting a blockage of the renaturation of the double-stranded nucleic acid; and
(v) determining the position of the blockage on the double-stranded nucleic acid molecule.

8. The method of any one of claims 1 to 7, wherein at least one of the bases of one of the strands of the double-stranded nucleic acid is attached directly or indirectly to a support, and wherein at least one of the bases of the other strand of the double-stranded nucleic acid is attached to a movable support.

9. The method of any one of claims 1 to 8, wherein the double-stranded nucleic acid is denatured in step (i) by moving away the supports, notably by applying a physical force above or equal to 15 pN, preferably above or equal to 17 pN, more preferably above or equal to 18 pN, to the double-stranded molecule by moving away the supports.

10. The method of any one of claims 1 to 9, wherein the denatured double-stranded nucleic acid is re-natured in step (iii) by bringing the supports together, notably by reducing the force applied to the double-stranded molecule to less than or equal to 12 pN, preferably less than or equal to 11 pN, more preferably less than or equal to 10 pN, by bringing the supports together.

11. The method of any one of claims 1 to 10, wherein the detection of step (iv) comprises measuring the distance (z) between the two ends of the double-stranded nucleic acid molecule which are attached to the support, measuring the distance (z_{high}) between the two ends of the double-stranded nucleic acid molecules which are attached to the support, when the said double-stranded nucleic acid molecule is denatured.

12. The method of claim 11, wherein step (v) further comprises the prior step of comparing z and z_{high}.

13. A method of quantifying a nucleic acid molecule in a population of nucleic acids comprising the steps of:
a) identifying the nucleic acid molecule using the method of any one of claims 1 to 12; and
B) numerating the nucleic acid molecule identified in step α).

14. A method of diagnosing a tumour in a patient, wherein the method comprises:
A. detecting the presence of a nucleic acid molecule in a sample from the patient using the method of any one of claims 1 to 12,
wherein said nucleic acid molecule comprises a tumour-specific signature.

15. The method of claim 14, further comprising the steps of:
B. quantifying a nucleic acid molecule comprising a tumour-specific signature using the method of claim 13;
wherein the presence of a tumour is indicated by a number of molecules in A.

16. The method of claim 15 further comprising the steps of:
C. quantifying a reference nucleic acid molecule using the method of claim 13; and
D. comparing the quantifications of steps A and B;
wherein the presence of a tumour is indicated by a number of molecules in A higher than the number of molecules in B.

17. The method of claim 16 wherein the number of molecules in A and the number of molecules in B are normalised to the total number of molecules.

18. The method of any one of claims 14 to 17 wherein the sample is selected in the group consisting of body fluids, including but not limited to blood, plasma, serum, mucus, lymphatic fluid, synovial fluid, cerebrospinal fluid, saliva, amniotic fluid, amniotic cord blood, urine, vaginal fluid and semen, preferably blood, serum and plasma.

19. The method of any one of claims 1 to 18 wherein the population of nucleic acid molecules is a population of cell-free DNA molecules.

20. A library of double stranded nucleic acid comprising at least one modified nucleotide triphosphate in one strand.
